(19) Europäisches Patentamt
European Patent Office
Office européen des brevets

(11) Publication number: 0 156 776 B1

(12) **EUROPEAN PATENT SPECIFICATION**

(45) Date of publication of patent specification: **29.03.89**

(51) Int. Cl.$^4$: **C 07 D 207/09**, C 07 C 65/21, C 07 C 101/72, A 61 K 31/40

(21) Application number: **85850022.6**

(22) Date of filing: **21.01.85**

(54) **Derivatives of N-(2-pyrrolidinylmethyl)-benzamide, process for their preparation, intermediates and a pharmaceutical preparation.**

(30) Priority: **31.01.84 SE 8400478**

(43) Date of publication of application:
**02.10.85 Bulletin 85/40**

(45) Publication of the grant of the patent:
**29.03.89 Bulletin 89/13**

(84) Designated Contracting States:
**AT BE CH DE FR IT LI LU NL SE**

(56) References cited:
**EP-A-0 004 831**
**EP-A-0 060 235**
**EP-A-0 117 384**
**CH-A- 639 369**

**JOURNAL OF MEDICINAL CHEMISTRY, 1982, vol. 25, no. 11, (1982), pp. 1980-1982**

**CHEMICAL ABSTRACTS, vol. 51, columns 12890i - 12891d, Columbus, Ohio, USA**

**CHEMICAL ABSTRACTS, vol. 38, column 3977 (7-9), Columbus, Ohio, USA**

(73) Proprietor: **Astra Läkemedel Aktiebolag**
**Strängnäsvägen 44**
**S-151 85 Södertälje (SE)**

(72) Inventor: **Bengtsson, Karl Stefan**
**Vadsbrovägen 12**
**S-153 00 Järna (SE)**
Inventor: **Högberg, Thomas**
**Vallmostigen 5**
**S-153 00 Järna (SE)**
Inventor: **Johansson, Lars George**
**Liljevalchsgatan 9**
**S-151 45 Södertälje (SE)**
Inventor: **De Paulis, Tomas**
**Nytropsvägen 24**
**S-144 00 Rönninge (SE)**
Inventor: **Ström, Hans Eric Peter**
**Parkvägen 3C-II**
**S-153 00 Järna (SE)**
Inventor: **Widman, Marianne Elisabet**
**Gripsvallsvägen 3**
**S-183 46 Täby (SE)**
Inventor: **Ögren, Sven Ove**
**Hökmossvägen 14B**
**S-155 00 Nykvarn (SE)**

(74) Representative: **Danielsson, Sten Ove et al**
**AB ASTRA Patent and Trademark Department**
**S-151 85 Södertälje (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 156 776 B1

Courier Press, Leamington Spa, England.

## Description

Field of the Invention

The present invention relates to novel, pharmacologically active derivatives of oxy-substituted salicylamides, intermediates, and processes for their preparation and pharmaceutical compositions containing the oxysalicylamido derivatives.

The object of the invention is to provide a substituted benzamide neuroleptic useful for the blockade of dopamine receptors in the brain. Such substances will be useful in the treatment of emesis, anxiety states, psychosomatic diseases and psychotic states, such as schizophrenia and depression, alcoholic related diseases, confusional states and sleep disturbances in the elderly.

Prior Art

Remoxipride (U.S. Patent No. 4 232 037) with the formula

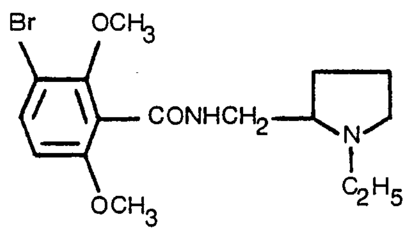

is a recently developed antipsychotic agent. This compound is claimed to be a potent antagonist of the apomorphine syndrome in the rat.

In European Patent Application No. 60235 benzamido derivatives claimed to be potent inhibitors of the apomorphine syndrome in the rat, are disclosed, among these the compound of the formula

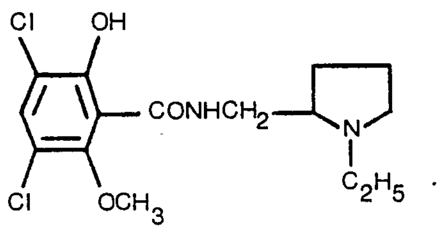

The compounds of U.S. 4 232 037 and EP 60235 have less potent antidopaminergic effects than the compounds of the present invention.

Disclosure of the Invention

The present invention relates to compounds of the formula

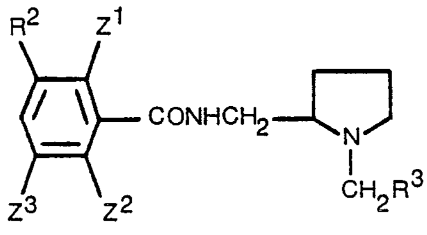

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C{-}(CH_2)_n{-}$,

$R^3$ is a hydrogen atom, $R^4$, a straight or branched hydrocarbon chain with a double or triple bond and 2 to 3 carbon atoms, phenyl or phenyl substituted with methylenedioxy or one or more of fluoro, chloro, bromo, trifluoromethyl, methyl, ethyl, methoxy or ethoxy in the orto, meta or para positions,

$R^1$ is alkyl CO, wherein alkyl represents a straight or branched hydrocarbon chain having 1 to 17 carbon atoms,

$R^4$ is an alkyl group of 1—4 carbon atoms,

n is 0, 1 or 2,

or a physiologically acceptable salt or optical isomer thereof.

It has been found that such compounds have valuable therapeutical properties, particularly they have more potent antidopaminergic effects than the prior art compounds discussed above and they also exhibit a larger separation to drug induced extrapyramidal side effects.

The invention thus provides compounds, and physiologically acceptable salts thereof, which compounds are useful in therapeutic treatment of emesis, anxiety states, phychosomatic diseases such as gastric and duodenal ulcer, and psychotic states such as schizophrenia and depression, alcoholic related diseases, confusional states and sleep disturbances in the elderly.

Halogen atoms in formula I comprise chlorine, bromine, fluorine and iodine atoms.

Lower alkyl groups in formula I are straight or branched alkyl groups with 1 to 4 carbon atoms, such as methyl, ethyl, n-propyl, i-propyl, n-butyl, s-butyl, i-butyl and t-butyl.

Lower trifluoroalkyl group in formula I is a group $F_3C-(CH_2)_n-$ wherein n is 0, 1 or 2.

Alkenyl groups in formula I are straight or branched hydrocarbon chains with 2 to 3 carbon atoms and a double bond, such as vinyl, allyl or isopropenyl.

Alkynyl groups in formula I are hydrocarbon chains with 2 to 3 carbon atoms with a triple bond, that is $-C{=}CH_3$, $-CH_2-C{\equiv}CH_3$ and $-C{\equiv}CCH_3$.

Acyl groups in formula I are a alkyl-CO— where the alkyl moiety is a straight or branched hydrocarbon chain with 1 to 17 carbon atoms, preferably 1—15 carbon atoms.

Phenyl substituted with methylenedioxy in formula I is the group

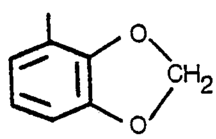

or

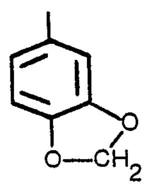

Preferred groups of compounds of the invention are obtained when in formula I

a) $Z^1$ is OH or O-acyl, $Z^2$ is OMe or θEt and $Z^3$ is OH, OMe, OEt, O-acyl, with one or both of $Z^2$ and $Z^3$ being OME or OEt,

$R^2$ is Cl, Br, I, Me, Et, Pr,

$R^3$ is hydrogen, methyl, ethyl, vinyl, ethynyl or substituted phenyl group as defined on p. 2.

b) $Z^1$ is OH or O-acyl, $Z^2$ is OMe or OEt and $Z^3$ is OH, OMe, OEt, O-acyl with one or both of $Z^2$ and $Z^3$ being OMe or OEt,

$R^2$ is Cl, Br, Et, Pr,

$R^3$ is as in a), or

c) $Z^1$ is OH or O-acyl, $Z^2$ is OME and $Z^3$ is OH, OMe and O-acyl with one or both of $Z^2$ and $Z^3$ being OMe,

$R^2$ is Cl, Br, Et, Pr,

$R^3$ is methyl, ethyl, vinyl or substituted phenyl, or

d) $Z^1$ is OH or O-acyl,

$Z^2$ and $Z^3$ is OME,

$R^2$ is Cl, Br, Et, Pr,

$R^3$ is methyl or vinyl and the configuration of the pyrrolidine ring being S (sinister) or phenyl or para-halogen substituted phenyl and the configuration of the pyrrolidine ring being R (rectus).

Compounds particularly preferred are

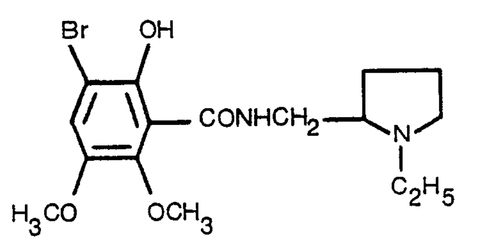

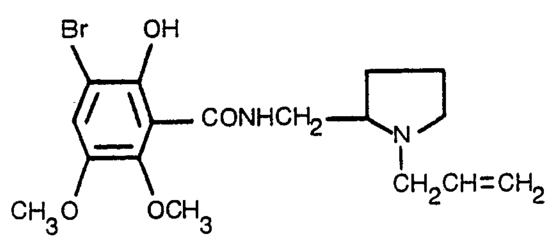

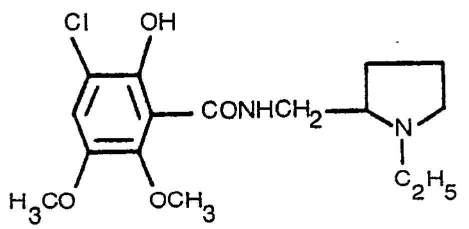

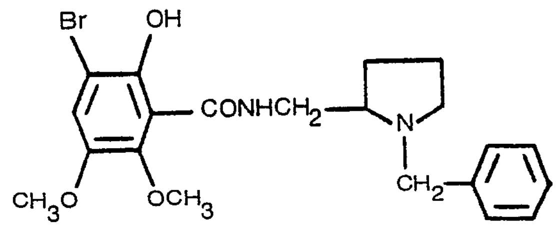

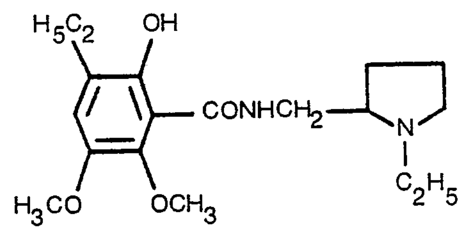

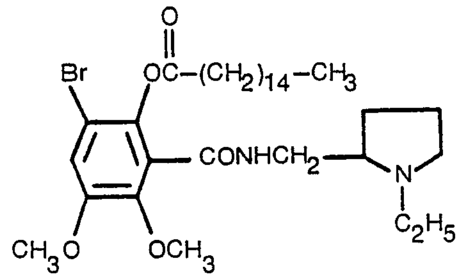

The new compounds of this invention may be used therapeutically as the racemic mixtures of (+)- and (−)-forms, which are obtained by synthesis. They may also be resolved into the corresponding enantiomers which, likewise, may be used in therapy. The (+)- and (−)-forms may also be obtained by the reaction of the corresponding enantiomeric 2-aminomethylpyrrolidine derivative with the benzoic acid moiety.

The compounds of this invention may be administered in the form of free bases or their salts with non-toxic acids. Some typical examples of these salts are the hydrobromide, hydrochloride, phosphate, sulphate, sulphonate, sulphamate, citrate, lactate, maleate, tartrate and acetate.

Pharmaceutical preparations

In clinical practice the compounds of the present invention will normally be administered orally, rectally or by injection in the form of pharmaceutical preparations comprising the active ingredient either as a free base or as a pharmaceutically acceptable non-toxic, acid addition salt, e.g. the hydrobromide, hydrochloride, phosphate, sulphate, sulphonate, sulphamate, citrate, lactate, maleate, tartrate, acetate and the like in association with a pharmaceutically acceptable carrier. Accordingly, terms relating to the novel compounds of this invention whether generically or specifically are intended to include both the free amine base and the acid addition salts of the free base, unless the context in which such terms are used, e.g. in the specific examples would be inconsistent with the broad concept.

The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparations intended for injection and between 2 and 50% by weight for preparations suitable for oral administration.

To produce pharmaceutical preparations containing a compound of the invention in the form of dosage units for oral application, the selected compound may be mixed with a solid pulverulent carrier, e.g. lactose, saccharose, sorbitol, mannitol, starches such as potato starch, corn starch or amylopectin, cellulose derivatives, or gelatine, and a lubricant such as magnesium stearate, calcium stearate, polyethylene glycol waxes, and the like, and then compressed to form tablets. If coated tablets are required, the cores, prepared as described above, may be coated with a concentrated sugar solution which may contain e.g. gum arabic, gelatine, talcum, titanium dioxide, and the like.

Alternatively, the tablet can be coated with a lacquer dissolved in a readily volatile organic solvent or mixture of organic solvents. Dyestuffs may be added to these coatings in order to readily distinguish between tablets containing different active substances or different amounts of the active compound.

For the preparation of soft gelatine capsules (pearl-shaped closed capsules) consisting of gelatine and for example, glycerol or similar closed capsules, the active substance may be admixed with a vegetable oil. Hard gelatine capsules may contain granulations of the active substance in combination with solid, powder carriers such as lactose, saccharose, sorbitol, mannitol, starches (e.g. potato starch, corn starch or amylopectin), cellulose derivatives or gelatine.

Dosage units for rectal application can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from about 0.2 to about 20% by weight of the active substance herein described, the balance being sugar and a mixture of ethanol, water, glycerol, and propyleneglycol. Optionally such liquid preparations may contain colouring agents, flavouring agents, saccharine and carboxymethylcellulose as a thickening agent.

Solutions for parenteral applications by injection can be prepared in an aqueous solution of a watersoluble pharmaceutically acceptable salt of the active substance preferably in a concentration of from about 0.5 to about 10% by weight. These solutions may also contain stabilizing agents and/or buffering agents and may conveniently be provided in various dosage unit ampoules.

Compounds of the formula I wherein $R^1$ is an acyl group may advantageously be used in pharmaceutical preparations intended for intramuscular administration in order to obtain a sustained release effect, that is a depot effect.

Suitable daily doses for oral administration of the compounds of this invention are 1—50 mg, preferably 5—20 mg.

Methods of Preparation

The compounds of the invention may be obtained by one of the following methods.

A. The compounds of the formula

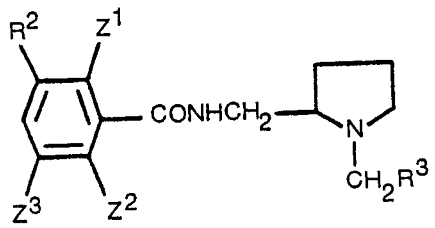

I

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C—(CH_2)_n$,

$R^3$ is a hydrogen atom, $R^4$, a straight or branched hydrocarbon chain with a double or triple bond and 2 to 3 carbon atoms, phenyl or phenyl substituted with methylenedioxy or one or more of fluoro, chloro, bromo, trifluoromethyl, methyl, ethyl, methoxy or ethoxy in the orto, meta or para positions,

$R^1$ is alkyl CO, wherein alkyl represents a straight or branched hydrocarbon chain having 1 to 17 carbon atoms,

$R^4$ is an alkyl group of 1—4 carbon atoms,

n is 0, 1 or 2,

can be obtained by reaction of a compound of the formula

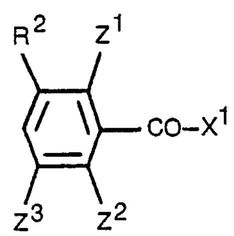

wherein $Z^1$, $Z^2$, $Z^3$ and $R^2$ have the above given definitions and $—CO—X^1$ is a reactive group capable of reacting with an amino group under formation of an amide moiety, with a compound of the formula

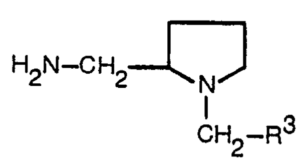

wherein $R^3$ has the above given definition, or a reactive derivative thereof.

The reaction is carried out in a suitable solvent, such as diethyl ether, THF, dichloromethane, chloroform or toluene between −20°C and the boiling point of the reaction mixture. The resulting amine can be isolated as a salt recovered e.g. by filtration. Alternatively, the amine obtained can be converted to

the free base using conventional techniques, such as the addition of aqueous ammonia or a sodium hydroxide solution, and extraction with an organic solvent.

$X^1$ in the acylating group —CO—$X^1$ may be a halogen group, such as chlorine or bromine, a mixed anhydride with inorganic acids or their esters, e.g. phenylphosphate, a thio group, an organic residue, or a hydroxy group in combination with a coupling agent or reactive amine derivative.

The organic residue comprises groups which can form reactive acid derivatives. These can be aliphatic esters, e.g. methyl, ethyl, cyanomethyl or methoxymethyl esters, N-hydroxyimide esters or substituted or unsubstituted aromatic esters; acyl nitrile; acyl azide; symmetrical anhydrides; mixed anhydrides; or azolides, e.g. triazolide, tetrazolide or imidazolide.

According to the invention the following compounds can be used as reactive derivatives of the cyclic amine above:

Reaction products of the amine with phosphorus chloride, phosphorus oxychloride, dialkyl, diaryl or o-phenylenechlorophosphites or alkyl or aryldichlorophosphites, or an isothiocyanate or isocyanate of the amine. The mentioned reactive derivatives can be reacted with the acid in situ or after previous isolation.

It is also possible to react the free acid and the free amine in the presence of a condensating agent, e.g. silicon tetrachloride, diphosphoruspentoxide, a phosphine or hexamethylphosphorus triamide plus a carbon tetrahalide, diphenyl phosphite, N-ethoxycarbonyl-2-ethoxy-1,2-dihydroquinoline, titanium tetrachloride, or carbodiimides such as dicyclocarbodiimide, N,N'-carbonyldiimidazole, N,N'-thionyl-diimidazole and diethyldiazodicarboxylate.

B. The compounds of the formula I, wherein $Z^1$, $Z^2$, $Z^3$, $R^2$ and $R^3$ are as defined in A can be obtained by N-substitution of a compound of the formula

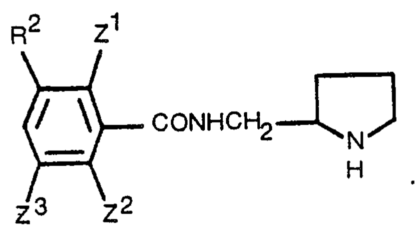

wherein $R^2$, $Z^1$, $Z^2$ and $Z^3$ have the definition given above, with a compound of the formula

$$R^3—CH_2—X^2$$

wherein $R^3$ has the definition given in A and $X^2$ is a leaving group, such as chlorine, bromine, sulphate, phosphate, benzenesulphonate or toluenesulphonate.

The reaction can be effected by treating the reactants at 0—100°C in a suitable solvent, e.g. acetone, alchols, dimethylformamide (DMF), dimethylsulphoxide (DMSO) in the presence of a base, for example NaOH or $K_2CO_3$.

C. The compounds of the formula

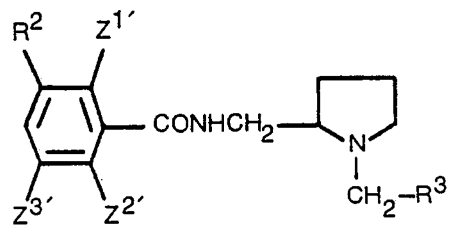

wherein $R^2$ and $R^3$ are as defined in A and $Z^{1'}$ is OH, $Z^{2'}$ is $OR^4$ and $Z^{3'}$ is OH or $OR^4$, can be obtained by reduction of a compound of the formula

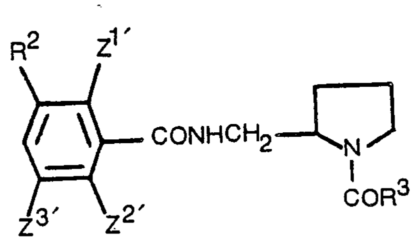

wherein $R^2$, $R^3$, $Z^{1'}$, $Z^{2'}$ and $Z^{3'}$ have the definitions given above.

Suitable reducing agents working on the less sterically hindered amide group are a) $LiAlH_4$ and alkoxy complexes thereof; b) $NaBH_4$ with addition of transition metal salts, or $AlCl_3$ or $BF_3$ or $POCl_3$ or carboxylic acids such as $CH_3COOH$ and $CF_3COOH$; c) $B_2H_6$.

The reaction is preferably effected in alkyl ethers such as diethylether, dimethoxyethane, diglyme, THF, dioxane, at temperatures from 0°C to reflux temperatures of the reaction mixtures.

D. The compounds of the formula

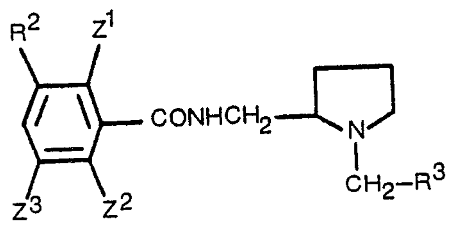

wherein $Z^1$, $Z^2$, $Z^3$, $R^2$ and $R^3$ are as defined in A and at least one of $Z^1$ and $Z^3$ is OH, can be obtained by deprotection of a compound of the formula

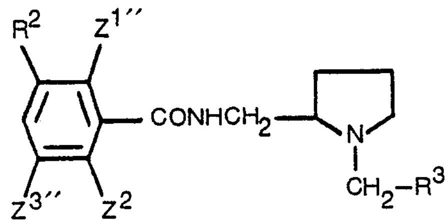

wherein $Z^2$, $R^2$ and $R^3$ are as defined in A and $Z^{1''}$ is OH, $OR^1$ or a suitably protected hydroxy group and $Z^{3''}$ is OH, $OR^4$, $OR^1$ or a suitably protected hydroxy group and at least one of $Z^{1''}$ and $Z^{3''}$ is a suitably protected hydroxy group.

Suitable standard phenol protective groups can be groups such as trimethylsilyl, t-butyldimethylsilyl, tetrahydropyranyl, benzyl, methoxyethoxymethyl, methoxymethyl, methylthiomethyl, aliphatic or aromatic esters, carbonates. The protective groups can be removed by standard procedures (T. W. Greene, in "Protective Groups in Organic Synthesis", Wiley, New York, 1981, p. 87—113).

A special form of protective group is represented by $Z^{1''}$ being $C_{1-4}$-alkoxy.

Thus, the compounds of the formula

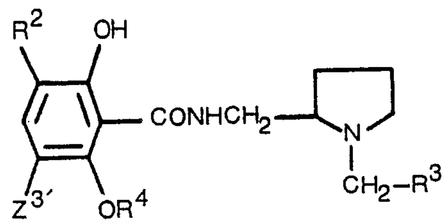

wherein $R^2$ and $R^3$ are as defined in A and $Z^{3'}$ is OH or $OR^4$, can be obtained by dealkylation of a compound of the formula

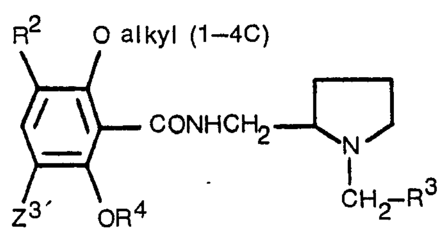

Suitable reagents are Brönsted acids (HBr, HI), Lewis Acids ($AlCl_3$, $AlBr_3$, $AlI_3$, $BBr_3$, $BCl_3$, 9-bromo-9-

borabicyclo[3.3.0]nonane, $NaBH_4/I_2$), nucleophilic reagents (sodium ethanethiolate, sodium phenylmethaneselenoate) and others (iodotrimethylsilane).

The reaction with Brönsted acids is performed at elevated temperatures preferably with a co-solvent like acetic acid or in the presence of a phase-transfer catalyst. The reaction with Lewis acids can be performed in refluxing benzene or carbon disulfide (aluminium halides) and halogenated solvents like dichloromethane at −75°C to 25°C (boron halides). Elevated temperature in dimethylformamide is suitable for nucleophilic reagents.

E. The compounds of the formula

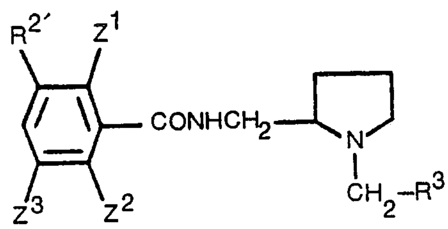

wherein $Z^1$, $Z^2$, $Z^3$ and $R^3$ are as defined in A and $R^{2'}$ is Br or Cl, can be obtained by reaction of a compound of the formula

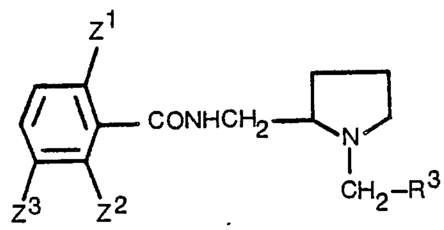

wherein $R^3$, $Z^1$, $Z^2$ and $Z^3$ have the above given definition, with a halogenating reagent such as halogen, a sulphurylhalogenide (preferably sulphurylchloride) or a halogen-dioxane complex.

Chlorination is effected by treating the starting compound with chlorine with or without Lewis acid catalysis or with sulphurylchloride, HOCl, N-chloroamides in the presence of acid catalyst in suitable solvent, e.g. chloroform, nitrobenzene.

Bromination is carried out with $Br_2$ with or without Lewis acid catalysis or bromination in acetic acid in the presence of a base e.g. sodium acetate or by using bromine-dioxane complex. Other reagents can be used among them HOBr and N-bromoamides especially N-bromosuccinimide with acid catalysis.

F. The compounds of the formula

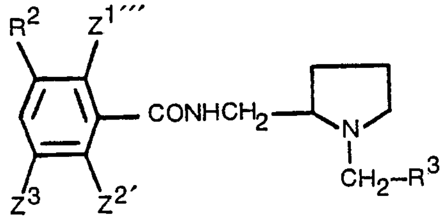

wherein $Z^3$, $R^2$ and $R^3$ are as defined in A and $Z^{1'''}$ and $Z^{2'}$ is $OR^4$, can be obtained by reaction of a compound of the formula

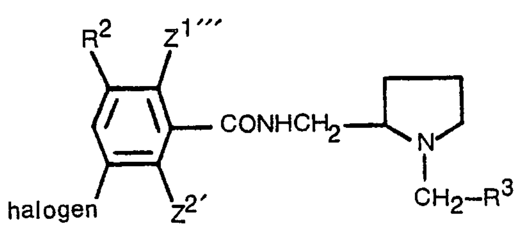

wherein $R^2$, $R^3$, $Z^{1'''}$ and $Z^{2'}$ are as defined above and halogen is e.g. Cl, Br or I, with potassium hydroxide or

sodium hydroxide in aqueous media, such as water in DMSO.

The reaction may be performed in water at 100°C in the presence of copper bronze or copper sulphate.

G. The compounds of the formula

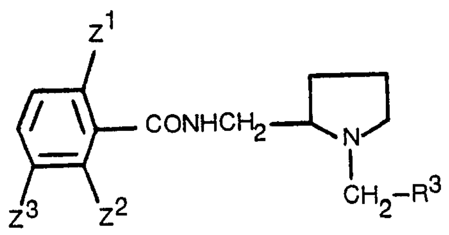

wherein $Z^1$, $Z^2$, $Z^3$ and $R^3$ are as defined in A, can be obtained by catalytic hydrogenation of a compound of the formula

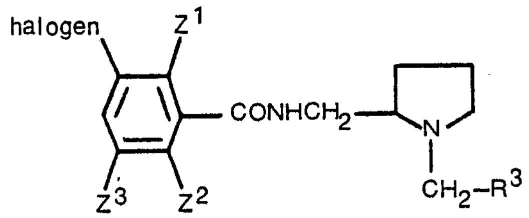

wherein $Z^1$, $Z^2$, $Z^3$ and $R^3$ are as defined above and halogen is e.g. Cl, Br or I.

The reaction is effected in a suitable solvent, e.g. methanol, ethanol.

H. The compounds of the formula

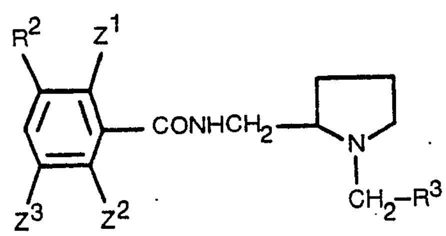

wherein $Z^1$, $Z^2$, $Z^3$, $R^2$ and $R^3$ are as defined in A and at least one of $Z^1$ and $Z^3$ is $OR^1$, can be obtained by reaction of a compound of the formula

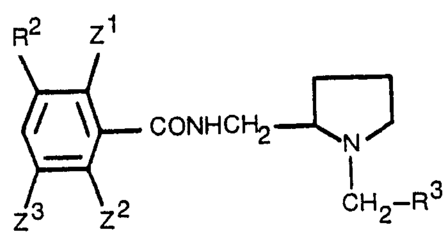

wherein $Z^1$, $Z^2$, $Z^3$, $R^2$ and $R^3$ are as defined above and at least one of $Z^1$ and $Z^3$ is OH, with a compound of the formula

$$R^1\text{—}X^3$$

wherein $R^1$ has the above given definition and $X^3$ is a suitable leaving group such as halogen (Cl, Br), acyloxy, azide or azolide without solvent or in a suitable solvent such as benzene or chloroform possibly with acid catalysis (e.g. $CF_3COOH$, $H_2SO_4$) or by using a tertiary amine as solvent and/or catalyst.

If desired, the compound obtained by any of methods A—H is converted to a physiologically acceptable salt thereof and/or converted to a substantially pure stereoisomer thereof.

Intermediates

The compounds of the formula

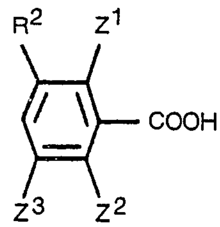

II

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C—(CH_2)_n—$,

$R^1$ is alkyl CO, wherein alkyl represents a straight or branched hydrocarbon chain having 1 to 17 carbon atoms,

$R^4$ is an alkyl group of 1—4 carbon atoms,

n is 0, 1 or 2,

are valuable intermediates for the preparation of the compounds of this invention by the process A.

Chemical Abstracts, Volume 38, column 3977 (7—9) and Chemical Abstracts, Volume 51, columns 12890; — 12891 d disclose compounds of the formula

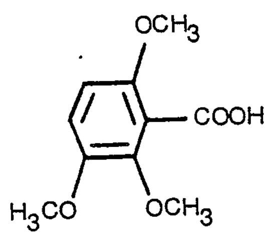

and

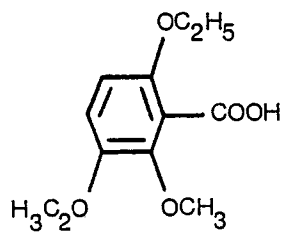

Compounds of the formula I wherein one, two or three of $Z^1$, $Z^2$ and $Z^3$ is a suitably protected phenol group are valuable intermediates for the preparation of deprotected compounds of the invention according to the process D.

The compounds of the formula

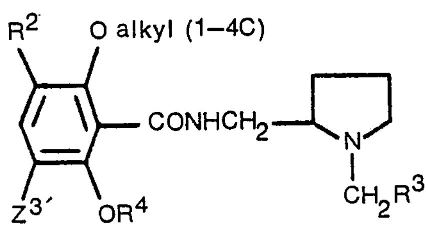

wherein $R^2$, $R^3$, $R^4$, and $Z^{3'}$ have the definition given under process D, are a subgroup within the group of compounds of the formula I, useful as intermediates for the preparation of dealkylated compounds of this invention.

The intermediates are prepared by the method described in A.

Intermediate of the formula

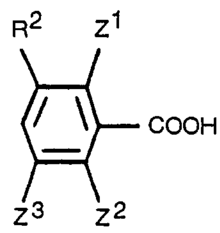

wherein $R^2$, $Z^1$, $Z^2$ and $Z^3$ are as defined above, may be prepared by

i) treating a compound of the formula

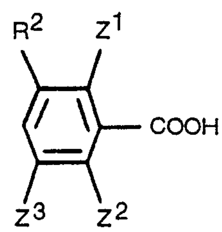

wherein $R^2$ is as defined above and $Z^1$, $Z^2$ and $Z^3$ are $OR^4$, wherein $R^4$ is as defined above, with a Lewis acid such as boron tribromide, boron trichloride or aluminum chloride or hydrobromic acid,

ii) treating a compound of the formula

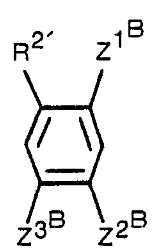

wherein $R^{2'}$ is as $R^2$ with the exception of Br and I; $Z^{1B}$, $Z^{2B}$ and $Z^{3B}$ being alkoxy; alternatively $Z^{1B}$, $Z^{2B}$ and $Z^{3B}$ can be a suitably protected derivative like methoxymethyl ether or tetrahydropyranyl ether, which is deprotected after the reaction, with alkyl- or aryllithium followed by reaction with carbon dioxide and acidification.

iii) treating a compound of the formula

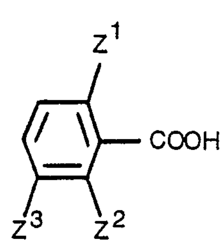

wherein $Z^1$, $Z^2$ and $Z^3$ are as defined previously, with halogen, a sulfurylhalogenide (preferably $SO_2Cl_2$) or a halogen-dioxane complex gives compounds with $R^2$ being halogen.

Working Examples

Example 1

(S)-(−)-N-[(1-ethyl-2-pyrrolidinyl)methyl]-3-bromo-2-hydroxy-5,6-dimethoxybenzamide

Method D

a) Compound (S)-(−)-N-[(1-ethyl-2-pyrrolidinyl)methyl]-3-bromo-2,5,6-trimethoxybenzamide (8.1 g, 0.020 mol) was dissolved in 100 ml $CH_2Cl_2$. 3—M HCl-ether (7.3 ml, 0.022 mol) was added at room

temperature followed by a solution of boron tribromide (5.5 g, 0.022 mol) in 40 ml $CH_2Cl_2$. After 1 h at 25°C 2—M ammonia (50 ml) was added, and the organic layer was separated, dried and evaporated. The residue (6.1 g) shows two peaks in GC with retention times 8.5 and 6.8 min, respectively, and two spots on TLC (silica in methanol-diisopropylether, 1:4) in the ratio 2:1. The major product was isolated by column chromatography to give 3.0 g of the title product. The hydrochloride was crystallized from 15 ml acetone-ether. M.p. 135—137°C.

Anal. ($C_{16}H_{24}BrClN_2O_4$): %C: calcd 45.35, found 45.22; %H: calcd 5.71, found 5.67; %N: calcd 6.61, found 6.56; %Br: calcd 18.86, found 18.75; %Cl: calcd 8.37, found 8.47.

$^1$H—NMR: ($CDCl_3$, δ ppm) 7.28 (s, 1H), 3.93 (s, 3H), 3.84 (s 3H), 3.70 (dd, 1H), 3.30 (m, 2H), 2.84 (dq, 1H), 2.6 (m, 1H), 2.20 (m, 2H), 1.4—1.8 (m, 4H), 1.13 (t, 3H).

$^{13}$H—NMR: aromatic region 169.2, 153.5, 147.9, 1444.6, 121.9, 109.0, 105.5.

b) From an anhydrous stock solution of (S)-N-[(1-ethyl-2-pyrrolidinyl)methyl]-3-bromo-5,6-dimethoxy-2-trimethylsilyloxybenzamide was withdrawn 0.5 mmol and treated with water at room temperature which caused rapid formation of the title compound which had identical NMR and GC retention time as the compound described in a).

Example 2

(S)-(−)-N-[(1-ethyl-2-pyrrolidinyl)methyl]-3-chloro-2-hydroxy-5,6-dimethoxybenzamide

Method D

To a solution of 2.0 g (S)-(−)-N-[(1-ethyl-2-pyrrolidinyl)methyl]-3-chloro-2,5,6-trimethoxybenzamide [0.0056 mol) and 1.9 ml 3M HCl-ether (0.0056 mol) in 20 ml $CH_2CL_2$, was a solution of 1.4 g $BBr_3$ in 10 ml $CH_2Cl_2$ added, over a period of 1 hour. After 1 hour at room temperature was the reaction mixture extracted with concentrated ammonia. The alkalified aqueous layer was extracted with 2 × 100 ml $CH_2Cl_2$. The organic layer was dried ($Na_2SO_4$) and evaporated. Gave a residue 1.3 g. TLC (silica in $iPr_2O$:MeOH-$NH_3$ 89:10:1) showed two spots Rf 0.45 and Rf 0.30, respectively. 0.9 g of the mixture was separated by column chromatography and gave 0.4 g of the title compound. The mesylate was crystallized from acetone. M.p. 165—166°C.

Anal. ($C_{16}H_{23}ClN_2O_4$): %C: calcd 46.52, found 46.53; %H: calcd 6.20, found 6.14; %Cl: calcd 8.08, found 7.89; calcd 6.38, found 6.30; %O: calcd 25.52, found 25.39; %S: calcd 7.31, found 7.38.

Example 3

(S)-(−)-N-[(1-ethyl-2-pyrrolidinyl)methyl]-3-ethyl-2-hydroxy-5,6-dimethoxybenzamide

Method D

A solution of (S)-(−)-N-[(1-ethyl-2-pyrrolidinyl)methyl]-3-ethyl-2,5,6-trimethoxybenzamide (0.80 g, 0.002 mol) in 25 ml $CH_2Cl_2$ was treated with 3—N HCl-ether (1 ml, 0.003 mol) followed by the addition of a solution of boron tribromide (0.6 g, 0.0023 mol) in 10 ml $CH_2Cl_2$ at ambient temperature. Work up and chromatography in accordance with example 9 gave 0.4 g (54%) of the title compound as an oil. Proton NMR: ($CDCl_3$) δ ppm: 9.2 (b, NH), 6.92 (s, $H_4$), 3.90 (s, $CH_3O$), 3.84 (s, $CH_3O$), 1.7—3.8 (m, 13H), 1.17 (t, $CH_3$), 1.13 (t, $CH_3$). Carbon-13 NMR: ($CDCl_3$) δ ppm: 170.2 CONH, 154.7 $C_2$—OH, 146.3 $C_6$—$OCH_3$, 143.8 $C_5$—$OCH_3$, 128.3 $C_3$—$C_2H_5$, 119.0 $Ch_4$—H, 107.5 $C_1$—CONH.

GC: Retention time 6.6 min. at 260°C on 10m SE 54. The minor isomer has RT 7.8 m.

The mesylate was prepared from ether by mixing one equivalent of methanesulfonic acid in acetone and recrystallizing from acetone. M.p. 153—155°C (acetone). Yield 0.32 g (38%).

Analysis ($C_{19}H_{32}N_2O_7S$): %C: calcd 52.76, found 52.69; %H: calcd 7.46, found 7.33; %N: calcd 6.48, found 6.44; %O: calcd 25.89, found 25.76; %S: calcd 7.41, found 7.27.

Example 4

(S)-(−)-5,6-Dimethoxy-N-[(1-ethyl-2-pyrrolidinyl)methyl]-2-hydroxy-3-propylbenzamide

Method D

A solution of 10.0 g (0.027 mol) of (S)-(−)-N-[(1-ethyl-2-pyrrolidinyl)methyl]-3-propyl-2,5,6-trimethoxybenzamide was treated with 16 ml (0.027 mol) of 1.6M HCl-ether in 250 ml of methylene chloride. A solution of 6.8 g (0.027 mol) of boron tribromide in 50 ml methylene chloride was slowly added at 10°C. The reaction mixture was stirred for 2 h at 20°C. 100 ml of 2M $NH_3$ was added. Extraction with 2 × 300 ml of $CH_2Cl_2$, drying ($Na_2SO_4$) and evaporation of the solvent gave 9.2 g of two components in a 4:1 ratio. The residue was dissolved in 300 ml of ether and shaken with 2 × 50 ml of 1N NaOH which exclusively removed the minor component from the ether layer. Drying and evaporation of the solvent gave 6.0 g of the title compound as an oil. GC 5.5 min at 250°C (SE—54). Yield 63%.

$^{13}$C—NMR ($CDCl_3$) δ 170.2 (CONH), 154.9 (C—2), 146.3 (C—6), 43.6 (C—5), 126.7 (C—3), 119.9 (C—4), 107.5 (C—1), 62.2 ($OCH_3$), 62.1 ($OCH_3$), 61.2 ($C^1$—2), 57.2, 53.4, 47.7, 40.5, 32.0, 28.4, 22.6; 14.0, 13.9 (9 carbons) ppm.

The oil was dissolved in 75 ml acetone. A hot solution of 2.6 g of L(+)-tartaric acid in 95 ml 98% (aq) acetone was added which gave 4.5 g of the tartrate salt. M.p. 84—85°C.

Example 5
(S)-(−)-N-[(1-Allyl-2-pyrrolidinyl)methyl]-3-bromo-2-hydroxy-5,6-dimethoxybenzamide
Method D

By the same method as described in Example 1, (S)-(−)-N-[(1-Allyl-2-pyrrolidinyl)methyl)-3-bromo-2,5,6-trimethoxybenzamide was transformed into the title compound. Yield 48% of colourless oil. $[\alpha]_D^{20}$ = −62° (c = 1.8, acetone).

$^1$HMR ($CDCl_3$): δ 9.05 (b, NH), 7.27 (s, D—4), 5.91 (m, vinyl-H), 5.19 (dd, 1H), 5.12 (d, 1H), 3.92 (s, $OCH_3$), 3.83 (s, $OCH_3$), 1.6—3.8 (m, 11H) ppm.

$^{13}$C—NMR ($CDCl_3$): δ 169.3 (CONH), 153.6 (C—2), 148.0 (C—6), 144.6 (C—5), 135.9 (CH—4), 122.0 (vinyl-CH), 117.0 (vinyl-$CH_2$), 1.09.1 (C—1), 105.6 (C—3), 61.5 (CH—2'), 61.4 ($OCH_3$—5), 57.2 ($OCH_3$—6), 56.9 ($NHCH_2$), 54.2 ($NCH_2$), 40.6 ($CH_2$—5'), 28.4 ($CH_2$—3'), 22.8 ($CH_2$—4') ppm.

Example 6
(R)-(+)-N-[(1-Benzyl-2-pyrrolidinyl)methyl]-3-bromo-2-hydroxy-5,6-dimethoxybenzamide
Method D

(R)-(+)-N-[(1-Benzyl-2-pyrrolidinyl)methyl]-3-bromo-2,5,6-trimethoxybenzamide (950 mg, 2.05 mmol) was dissolved in 30 ml dichloromethane and cooled with ice. Solutions of 4M HCl in ether (0.5 ml, 2 mmol) followed by 3.2 ml 0.65M boron tribromide in dichloromethane (2.1 mmol) were added. After stirring for 1 h 30 ml 0.7M $NH_3$ were added, the mixture extracted with dichloromethane. The solvent was evaporated, the residue dissolved in $Et_2O$, washed with brine, dried ($MgSO_4$) and evaporated to give 923 mg (100%) of two isomeric phenols. GC (SE 30, capillary column, 270°C): retention times 10.1 min and 12.4 min (ratio 3:7). The phenols were separated by flash chromatography on $SiO_2$ with $Et_2O$/MeOH/$NH_3$ 100:3:0.3 to give 495 mg (54%) of the total compound as an oil.

$[\alpha]_D^{22}$ = +94° (c = 0.52, acetone). $^1$H—NMR ($CDCl_3$): 7.25 (s, overlapping with Ph, 4—H), 3.81 and 3.75 (two s, $(OMe)_2$). Mass spectrum (EI, 70 ev): m/z 449/447 (M—H, 0.57/0.61%), 261/259 (ArCO, 1.3/1.3%), 160 (100%), 91 (51%).

Example 7
(S)-(−)-N-[(1-Ethyl-2-pyrrolidinyl)methyl]-5,6-Dimethoxy-2-hydroxybenzamide hydrochloride
Method G

A solution of (S)-(−)-3-bromo-N-[(1-ethyl-2-pyrrolidinyl)methyl]-2-hydroxy-5,6-dimethoxybenzamide HCl (0.20 g, 0.47 mmol) in 10 ml of 95% ethanol was hydrogenated for 2.5 h at ambient pressure and temperature with 10 mg of palladium on charcoal as the catalyst. Filtration and evaporation of the solvent gave 0.18 g of the title products as an oil. Its chromatographic and spectroscopic properties (TLC, GC, NMR) were identical of those of the minor product obtained from the boron tribromide demethylation of (S)-(−)-N-[(1-ethyl-2-pyrrolidinyl)methyl]-2,5,6-trimethoxybenzamide.

Gas chromatography (GC): Retention time at 230°C on 25 m SE—52 in 2.50 min (72% of that of its isomer).

NMR: proton ($CDCl_3$): δ ppm 8.4 (b, NH), 7.02 (d, J=9.15Hz, $H_4$), 6.70 (d, $H_3$), 3.93 (s, $CH_3O$), 3.83 (s, $CH_3O$), 1.7—3.8 (m, 11H), 1.13 (t, $CH_3CH_2$).

NMR: carbon-13 ($CDCl_3$) δ ppm: 169.8 CONH, 156.9 $C_2$—OH, 148.3 $C_6$—OMe, 144.4 $C_5$—OMe, 118.9 $C_4$—H, 113.1 $C_3$—H, 108.3 $C_1$—CONH.

Example 8
(S)-(−)-N-[(1-Ethyl-2-pyrrolidinyl)methyl]-3-bromo-2-hydroxy-5,6-dimethoxybenzamide
Method E

(S)-N-[(1-Ethyl-2-pyrrolidinyl)methyl]-2-hydroxy-5,6-dimethoxybenzamide (140 mg, 0.45 mmol) was dissolved in 5 ml dioxane. After addition of 0.1 g $K_2CO_3$ a solution of 30 μl $Br_2$ in 2 ml dioxane was added. After stirring for 2 h the mixture was partitioned between 2M $NH_3$ and $Et_2O$. Drying ($MgSO_4$) and evaporation of the organic layer gave 165 mg (95%) of the title compound having identical NMR and GC retention time as the compound prepared in Example 1 .

Example 9
(S)-N-[(1-Ethyl-2-pyrrolidinyl)methyl]-3-bromo-2-hexadecanoyl-5,6-dimethoxybenzamide
Method H

To a solution of (S)-N-[(1-ethyl-2-pyrrolidinyl)methyl]-3-bromo-2-hydroxy-5,6-dimethoxybenzamide mesylate (0.48 g, 1 mmol) in 10 ml of trifluoroacetic acid was added palmitoyl chloride (0.55 ml, 2 mmol). The reaction mixture was stirred at ambient temperature over molecular sieves for 20 h. After evaporation of the solvent in vacuo ether was added to the residue and filtered. The filtrate was washed several times with a saturate $KHCO_3$ solution and dried ($MgSO_4$). After evaporation of the solvent in vacuo the resulting oil crystallized on cooling. Yield 0.42 g (67%). M.p. 46—48°C. $R_f$ value is 0.21 for title compound and 0.32 for starting compound ($SiO_2$, TLC-plates, 20% MeOH in i-$Pr_2O$ as eluant). Mass spectrum (EI, 79eV) m/z 624/626 $M^+$).

Example 10

(S)-(−)-N-[(1-Ethyl-2-pyrrolidinyl)methyl]-2,5-dibenzyloxy-6-methoxybenzamide

Method A

A solution of 3,6-dibenzyloxy-2-methoxybenzoic acid (120 mg, 0.33 mmol), thionyl chloride (120 mg, 1 mmol) and two drops of dimethylformamide as catalyst in 5 ml toluene was stirred at 60° for 1.5 h. The solvent was evaporated and the residue dissolved in $CH_2Cl_2$ and evaporated again. This residue was dissolved in 8 ml $CH_2Cl_2$ and a solution of (S)-(−)-1-ethyl-2-aminomethylpyrrolidine (65 mg, 0.5 mmol) in 2 ml $CH_2Cl_2$ was added. After stirring overnight at room temperature the solvent was evaporated and the residue partitioned between 2M HCl and ether. The aqueous phase was made alkaline, extracted with $CH_2Cl_2$, dried ($Na_2SO_4$) and evaporated to give a crude product. Purification by chromatography on $SiO_2$ with $iPr_2O$/hexane/MeOH/$NH_3$ 69:20:10:1 as eluent gave 145 mg (93%) pure title compound. M.p. 121—123°C. $[\alpha]_D^{22} = -42°$ (c = 2.8, acetone).

$^1H$ NMR ($CDCl_3$) δ 7.39 and 7.38 (two s, $CH_2$*Ph*), 6.89 and 6.59 (AB, 4—H, and 5—H), 5.06 and 5.03 (two s, *$CH_2$*Ph), 3.95 (s, OMe) ppm. Mass spectrum (EI, 70 eV): m/z 474 (M, 0.13%), 347 (ArCO, 0.33%), 98 (100%), 91 (12%).

Example 11

(S)-(−)-[(1-Ethyl-2-pyrrolidinyl)methyl]-2,5-dihydroxy-6-methoxybenzamide

Method D

A mixture of (S)-(−)-[(1-ethyl-2-pyrrolidinyl)methyl]-2,5-dibenzyloxy-6-methoxybenzamide (130 mg, 0.27 mmol), 5% Pd/C (50 mg), 0.5 ml 4M HCl in ether and 5 ml ethanol was shaken in a hydrogen atmosphere for 1 h. Filtration and evaporation of the solvent gave 90 mg pure title compound as an oily hydrochloride.

$^1H$ NMR ($CDCl_3$/$CD_3OD$): δ 7.13 and 6.67 (AB, 4—H and 5—H), 3.99 (s, OME) ppm.

Mass spectrum (EI, 70 eV): m/z 294 (M, 0.64%), 167 (ArCO, 1.4%), 98 (100%).

Example 12

(S)-(−)-[(1-ethyl-2-pyrrolidinyl)methyl]-3-bromo-2,5-dihydroxy-6-methoxybenzamide

Method E

To a mixture of (S)-(−)-[(1-ethyl-2-pyrrolidinyl)methyl]-3,6-dihydroxy-2-methoxybenzamide hydrochloride (90 mg, 0.27 mmol), 0.5 dioxane and 0.1 ml acetic acid was added a solution of 18 µl bromine (0.35 mmol) in 0.5 ml dioxane. After stirring at room temperature for 1 h the solvent was evaporated.

$^1H$ NMR ($CDCl_3$) showed complete removal of the aromatic AB system: δ 7.40 (s, 4—H), 4.05 (s, OMe) ppm.

Mass spectrum (EI, 70 eV): m/z 374/372 (M, 0.19%/0.18%), 247/245 (ArCO, 0.40%/0.40%), 98 (100%).

Example 13

By any of the methods described in the preceding examples the following compound can be prepared:

(S)-(−)-N-[(1-ethyl-2-pyrrolidinyl)methyl]-2-hydroxy-5,6-dimethoxy-3-methylbenzamide,

(S)-(−)-N-[(1-ethyl-2-pyrrolidinyl)methyl]-3-ethyl-2,5-dihydroxy-6-methoxybenzamide,

(S)-(−)-N-[(1-ethyl-2-pyrrolidinyl)methyl]-3-bromo-5,6-diethoxy-2-hydroxybenzamide.

Example 14

The following examples illustrate the preparation of pharmaceutical compositions of the invention. The wording "active substance" denotes a compound according to the present invention or a salt thereof.

Formulation A. Soft gelatin capsules

500 g of active substance were mixed with 500 g of corn oil, whereupon the mixture was filled in soft gelatin capsules, each capsule containing 100 mg of the mixture (i.e. 50 mg of active substance).

Formulation B. Soft gelatin capsules

500 g of active substance were mixed with 750 g of pea nut oil, whereupon the mixture was filled in soft gelatin capsules, each capsule containing 125 mg of the mixture (i.e. 50 mg of active substance).

Formulation C. Tablets

50 kg of active substance were mixed with 20 kg of silicic acid of the trademark Aerosil. 45 kg of potato starch and 50 kg of lactose were mixed therewith and the mixture was moistened with a starch paste prepared from 5 kg of potato starch and distilled water, whereupon the mixture was granulated through a sieve. The granulate was dried and sieved, whereupon 2 kg of magnesium stearate was mixed into it. Finally the mixture was pressed into tablets each weighing 172 mg.

Formulation D. Effervescing tablets

100 g of active substance, 140 g of finely divided citric acid, 100 g of finely divided sodium hydrogen carbonate, 3.5 g of magnesium stearate and flavouring agents (q.s.) were mixed and the mixture was pressed into tablets each containing 100 mg of active substance.

Formulation E. Sustained release tablet

200 g of active substance were melted together with 50 g of stearic acid and 50 g of carnauba wax. The mixture thus obtained was cooled and ground to a particle size of at most 1 mm in diameter. The mixture thus obtained was mixed with 5 g of magnesium stearate and pressed into tablets each weighing 305 mg. Each tablet thus contains 200 mg of active substance.

Formulation F. Injection solution

| | |
|---|---|
| Active substance | 3.000 mg |
| Sodium pyrosulfite | 0.500 mg |
| Disodium edetate | 0.100 mg |
| Sodium chloride | 8.500 mg |
| Sterile water for injection ad | 1.00 ml |

Formulation G. Hard gelatin capsules

10 g of active substance was mixed with 400 g of lactose and finally 2 g of magnesium stearate was added. The mixture was then filled in hard gelatin capsules, each capsule containing 206 mg of the mixture (i.e. 5 mg of active substance).

Formulation H. Tablets

50 g of active substance was mixed with 1500 g of lactose, 200 g of microcrystalline cellulose and 10 g magnesium stearate. Tablets of 5 mg active substance with a core weight of 176 mg were finally comprotted.

Formulation I. Depot preparation

| | | |
|---|---|---|
| (S)-N-[(1-Ethyl-2-pyrrolidinyl)methyl]-3-bromo-2-hexadecanoyl-5,6-dimethoxybenzamide | | 200 mg |
| Peanut oil | ad | 1 ml |

Pharmacology

Introduction

A number of studies suggest that the antipsychotic action of neuroleptic drugs is in some way related to the decrease in catecholamine transmission in the brain caused by these drugs and more specifically due to central dopamine (DA) receptor blockade in cortical and subcortical brain regions. Most compounds with an antipsychotic action affect several DA systems in the brain. There is evidence that the antipsychotic action may be linked to blockade of DA receptors in the subcortical and cortical limbic structures (J. Pharm. Pharmacol. *25*, 346, 1973; Lancet, 1027, 1976) while the wellknown extrapyramidal side effects produced by neuroleptic drugs are due to blockade of DA receptors in the nigroneostriatal DA system (Intern. J. Neurol. *6*, 27—45, 1967).

A. In vivo tests

There are presently several techniques available to study DA receptor blockade in the brain in vivo. One method is based on the ability of antipsychotic drugs to block the behavioural effects induced by the DA agonist apomorphine in the rat. Several studies indicate an excellent correlation between the in vivo DA receptor blockade as measured in the apomorphine test and therapeutic efficacy of different antipsychotic drugs. Apomorphine produces in rats and other species a characteristic syndrome consisting of repetitive movements (stereotypies) and hyperactivity which appear to be due to activation of postsynaptic DA receptors in the brain (J. Pharm. Pharmacol. *19*, 627, 1967; J. Neurol. Transm. *40*, 97—113, 1977). The stereotypies (chewing, licking, biting) appear mainly to be induced via activation of DA receptors linked to the nigro neostriatal DA system (J. Psychiat. Res., *11*, 1, 1974) whereas the increase locomotion (hyperactivity) mainly appears to be due to activation of DA receptors in subcortical mesolimbic structures (nucleus olfactorium, nucleus accumbens) i.e. the mesolimbic DA system. (J. Pharm. Pharmacol. *25*, 1003, 1973).

A number of studies have demonstrated that neuroleptics of different structural classes block the apomorphine stereotypies in the rat and that this blockade is well related to blockade of DA transmission measured by biochemical or neurophysiological techniques. Thus, the antiapomorphine effect correlates well with changes in DA turnover produced by neuroleptic drugs (Eur. J. Pharmacol., *11*, 303, 1970), DA

receptor binding studies (Life Science, *17*, 993—1002, 1976) and most important with antipsychotic efficacy (Nature, *263*, 388—341, 1976).

Methods

Male Sprague-Dawley rats (weighing 225—275 g) were used. The rats were observed in perspex cages (40 (L) × 25 (w) × 30 (h) cm) and the behaviour was scored 5, 20, 40 and 60 min. after apomorphine. The compounds were injected 60 min. prior to apomorphine hydrochloride (1 mg/kg) which was injected subcutaneously (s.c.) into the neck. This dose and form of administration was found to produce a very consistent response and very low variation in response strength. Furthermore, apomorphine given s.c. also produced a very consistent hyperactivity.

Direct after injection, the animals were placed in the cages, one in each cage. Scoring of the stereotypies were performed by two separate methods. The first scoring system was a modified version of the system introduced by Costall and Naylor (1973). The strength of the stereotypes was scored on a 0—3 scale as follows:

| Score | Description of stereotyped behaviour |
|---|---|
| 0 | No change in behaviour compared to saline controls or sedated |
| 1 | Discontinuous sniffing |
| 2 | Continuous sniffing |
| 3 | Continuous sniffing. Chewing, biting and licking. |

In the second system the number of animals displaying hyperactivity caused by apomorphine were scored. Each group consisted of 6—8 animals. Saline controls were always run simultaneously. $ED_{50}$'s are in the first scoring system (0—3 scale), the doses which reduce the strength of the stereotypies by 50% over the observation period of 60 min. $ED_{50}$'s of the second scoring system are the doses which reduce the number of animals showing hyperactivity by 50% over the observation period of 60 min. The $ED_{50}$ were calculated from log dose-response curves by the method of least squares from 4—6 dose levels with 6—8 animals per dose level.

*B: In vitro test: Receptor binding assay*

The clinical efficacy of antipsychotic drugs has been shown to correlate with their ability to displace tritiated spiperone from preparations of dopamine receptors (Seeman, Biochem. Pharmacol. *26*, 1741 (1977).

Method

The method of Burt et al. (Proc. Nat. Acad. Sci. USA *72*, 4655 (1975) was used. Male Sprague-Dawley rats weighing 150—200 g were decapitated, and their brains were rapidly removed. The striata were dissected, pooled and homogenized in 50 mM Tris-HCl buffer (pH 7.6). The membrane fraction was collected by centrifugation (48000 g for ten minutes), washed once with the buffer, and resuspended in 50 mM Tris-HCl (pH 7.6) containing 0.1% ascorbic acid, 10 mM pargyline, 120 mM NaCl, 5 mM KCl, 2 mM $CaCl_2$ and 1 mM $MgCl_2$. The suspension was preincubated at 37°C for 10 minutes and then kept on ice until use.

The assays have been carried out using a cell harvester equipment. The incubations were made in quadruplicate, each well containing membrane suspension (2.5 mg/0.5 ml), $^3$H-spiperone (0.4 mM) and the test compound in a final volume of 0.5 ml. After incubation for 10 minutes at 37°C, the contents of the wells were rapidly filtered and washed on Whatman GF/B filters using the Cell harvester. The specific binding was defined as the difference of ligand bound in the presence and in the absence of 1 µM (+)-butaclamol. The test results are expressed as IC50. The IC50 value given in µM, indicates the concentration of the test substance which reduces the amount of specifically bound spiperone by 50%.

Test results

The test results are given in the following table.

| Test compound | In vivo | | In vitro |
|---|---|---|---|
| | Reduction of stereotypes ED50 (µmole/kg i.p.) | Reduction of hyperactivity ED50 (µmole/kg i.p.) | Block of $^{3}H$-spiperone binding IC50 (µm) |
| Prior art compounds: | | | |
| 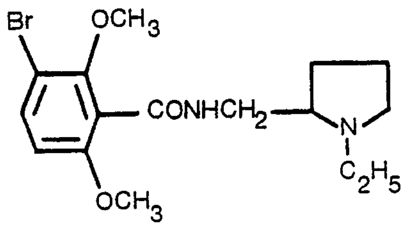 (remoxipride, US patent 4 232 037) | 6.5 | 0.86 | 1.57 |
| 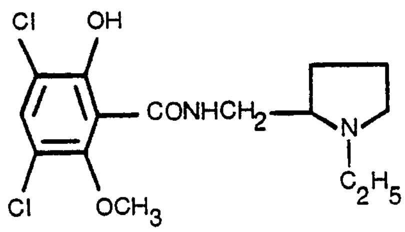 (a compound of EP 60235) | 2.4 | 0.11 | 0.026 |
| Compounds of the invention: | | | |
| 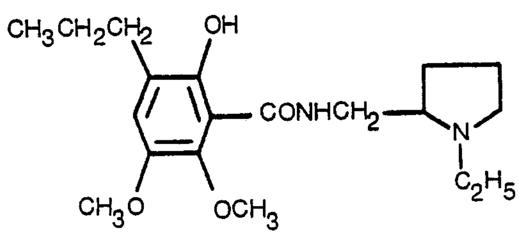 | $26.10^{-2}$ | $<10.10^{-3}$ | $24.10^{-4}$ |
| 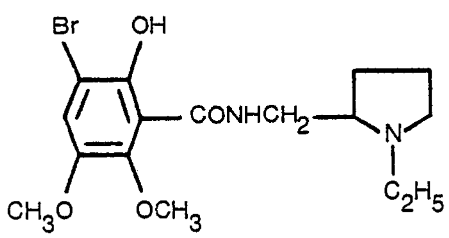 | $4.2\cdot10^{-2}$ | $5\cdot10^{-3}$ | $10\cdot10^{-4}$ |
| 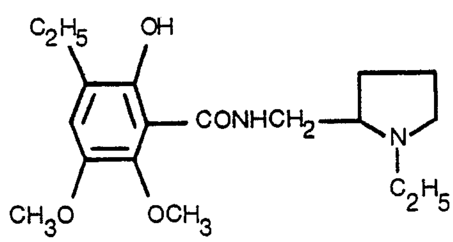 | $5.8\cdot10^{-2}$ | $0.7\cdot10^{-3}$ | $7\cdot10^{-4}$ |

Comments to the test results

The compounds of this invention exhibit an antidopamine activity superior to that of the tested compounds of the prior art both in vivo and in vitro. In their ability to inhibit the stereotypies induced by apomorphine in rats, the tested compounds of the invention are about 50—150 times more potent than the tested prior art compounds. Moreover, the difference between the $ED_{50}$ doses which block apomorphine-induced hyperactivity and $ED_{50}$ doses which block stereotypies is high, which indicates a highly selective action on specific dopamine neurons. These properties could not be predicted from the properties of the prior art compounds.

The receptor binding studies in vitro confirm the high potency found in vivo of the compounds of the invention. The activity of the compounds of the invention on displacement of $^3$H-spiperone from striatial preparations of the rat brain is very much higher than the activity of the tested prior art compounds.

**Claims for the Contracting States: BE CH DE FR IT LI LU NL SE**

1. A compound of the formula

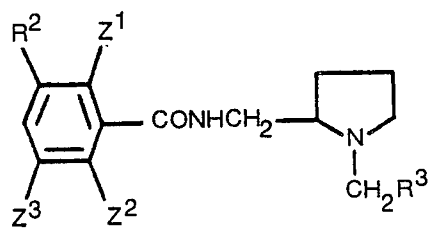

I

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C—(CH_2)_n—$

$R^3$ is a hydrogen atom, $R^4$, a straight or branched hydrocarbon chain with a double or triple bond and 2 to 3 carbon atoms, phenyl or phenyl substituted with methylenedioxy or one or more of fluoro, chloro, bromo, trifluoromethyl, methyl, ethyl, methoxy or ethoxy in the orto, meta or para positions,

$R^1$ is alkyl CO, wherein alkyl represents a straight or branched hydrocarbon chain having 1 to 17 carbon atoms,

$R^4$ is an alkyl group of 1—4 carbon atoms,

n is 0, 1 or 2,

or a physiologically acceptable salt or optical isomer thereof.

2. A compound according to claim 1 of the formula

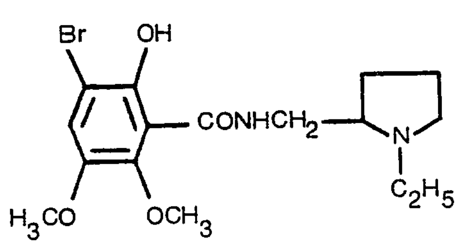

or

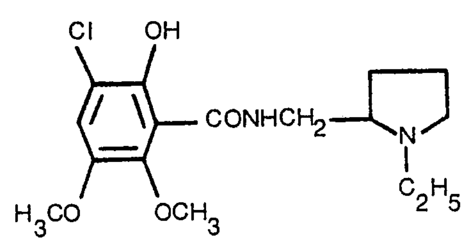

or

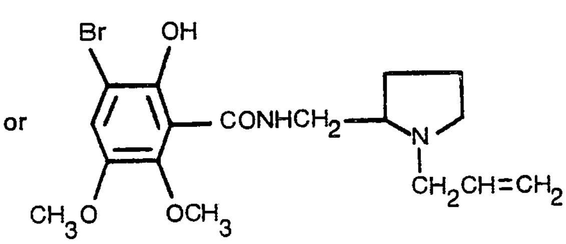

or

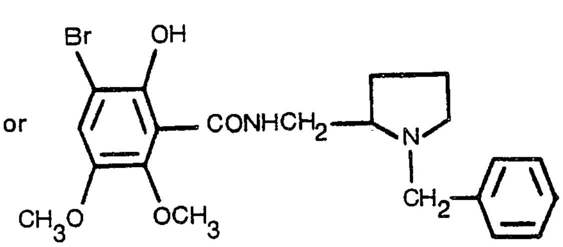

or 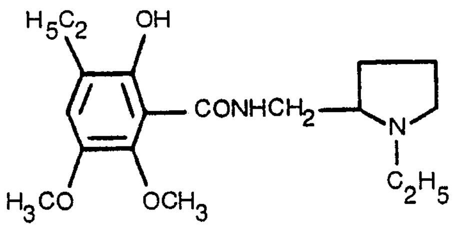 or 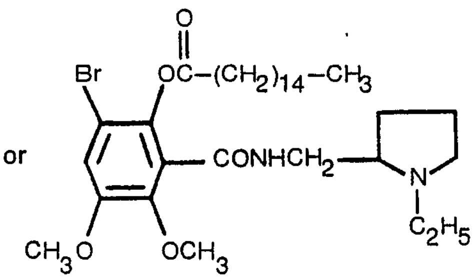

3. A process for the preparation of a compound of the formula

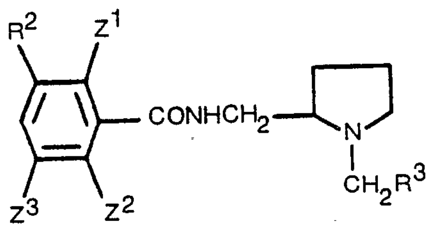 I

wherein
$Z^1$ is OH or $OR^1$,
$Z^2$ is $OR^4$,
$Z^3$ is OH, $OR^4$ or $OR^1$,
$R^2$ is a hydrogen atom, a halogen atom, $R^4$ or $F_3C—(CH_2)_n—$
$R^3$ is a hydrogen atom, $R^4$, a straight or branched hydrocarbon chain with a double or triple bond and 2 to 3 carbon atoms, phenyl or phenyl substituted with methylenedioxy or one or more of fluoro, chloro, bromo, trifluoromethyl, methyl, ethyl, methoxy or ethoxy in the orto, meta or para positions,
$R^1$ is alkyl CO, wherein alkyl represents a straight or branched hydrocarbon chain having 1 to 17 carbon atoms,
$R^4$ is an alkyl group of 1—4 carbon atoms,
n is 0, 1 or 2,
or a physiologically acceptable salt or optical isomer thereof, which process comprises
a) reaction of a compound of the formula

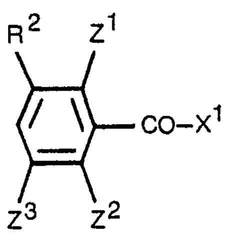

wherein $Z^1$, $Z^2$, $Z^3$ and $R^2$ have the above given definitions and $—CO—X^1$ is a reactive group capable of reacting with an amino group under formation of an amide moiety, with a compound of the formula

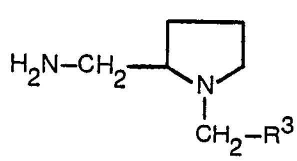

wherein $R^3$ has the above given definition, or a reactive derivative thereof, to the formation of a compound of the formula I, or

b) N-substitution of a compound of the formula

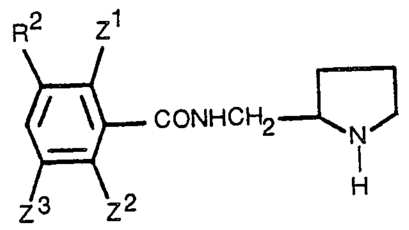

wherein $R^2$, $Z^1$, $Z^2$ and $Z^3$ have the definitions given in the preamble of the claim, with a compound of the formula

$$R^3—CH_2—X^2$$

wherein $R^3$ has the definition given in the preamble of the claim and $X^2$ is a leaving group, to the formation of a compound of the formula I, or

c) reduction of a compound of the formula

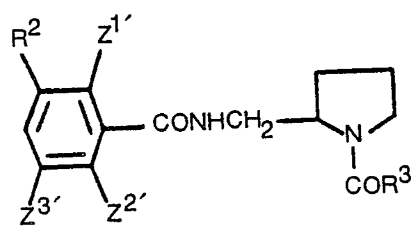

wherein $R^2$, $R^3$, $Z^{1'}$, $Z^{2'}$ and $Z^{3'}$ have the definitions given below, to the formation of a compound of the formula

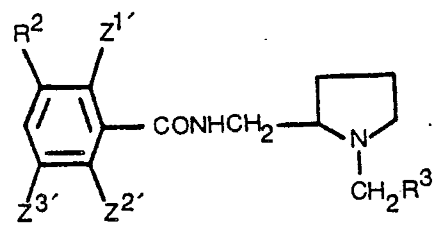

wherein $R^2$ and $R^3$ are as defined in the preamble of the claim and $Z^{1'}$ is OH, $Z^{2'}$ is $OR^4$ and $Z^{3'}$ is OH or $OR^4$, or

d) deprotection of a compound of the formula

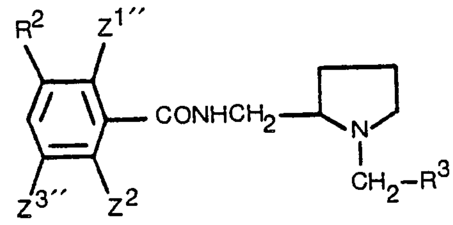

wherein $Z^2$, $R^2$ and $R^3$ are as defined in the preamble of the claim, $Z^{1''}$ is OH, $OR^1$ or a suitably protected hydroxy group and $Z^{3''}$ is OH, $OR^4$, $OR^1$ or a suitably protected hydroxy group and at least one of $Z^{1''}$ and $Z^{3''}$ is a suitably protected hydroxy group, to the formation of a compound of a formula

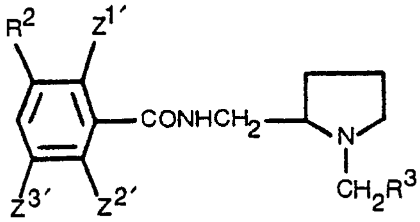

wherein $Z^1$, $Z^2$, $Z^3$, $R^2$ and $R^3$ are as defined in the preamble of the claim and at least one of $Z^1$ and $Z^3$ is OH, or

e) reaction of a compound of the formula

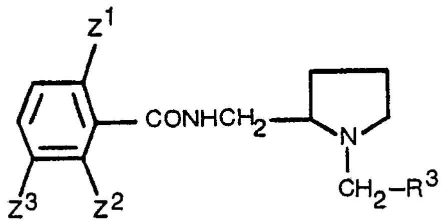

wherein $Z^1$, $Z^2$, $Z^3$ and $R^3$ are as defined in the preamble of the claim, with a halogenating agent, to the formation of a compound of the formula

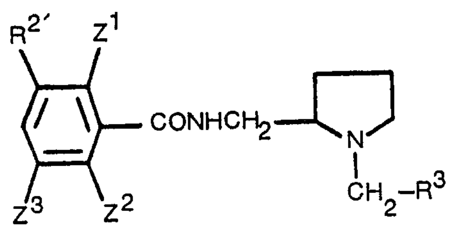

wherein $Z^1$, $Z^2$, $Z^3$ and $R^3$ are as defined in the preamble of the claim and $R^{2'}$ is Br or Cl, or

f) reaction of a compound of the formula

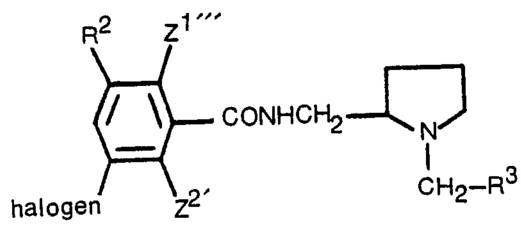

wherein $R^2$ and $R^3$ are as defined in the preamble of the claim, $Z^{1'''}$ and $Z^{2'}$ is $OR^4$ and halogen is Cl, Br or I, with potassium hydroxide or sodium hydroxide in aqueous media, to the formation of a compound of the formula

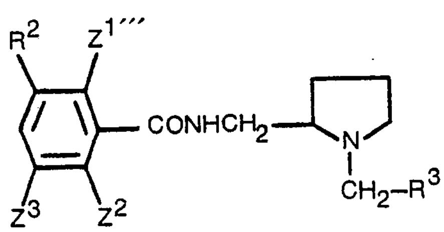

wherein $Z^3$, $R^2$ and $R^3$ are as defined in the preamble of the claim and $Z^{1'''}$ and $Z^{2'}$ is $OR^4$, or

g) catalytic hydrogenation of a compound of the formula

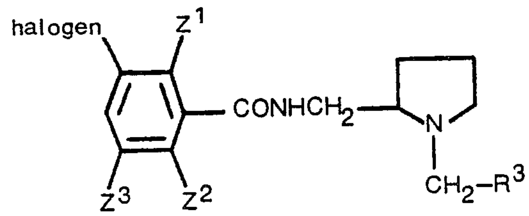

wherein $Z^1$, $Z^2$, $Z^3$ and $R^3$ are as defined in the preamble of the claim and halogen is Cl, Br or I, to the formation of a compound of the formula

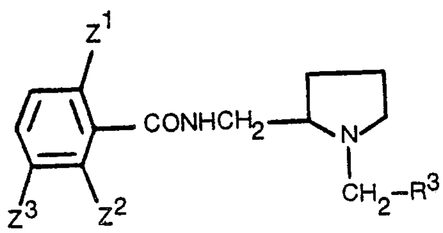

wherein $Z^1$, $Z^2$, $Z^3$ and $R^3$ are as defined in the preamble of the claim, or

h) reaction of a compound of the formula

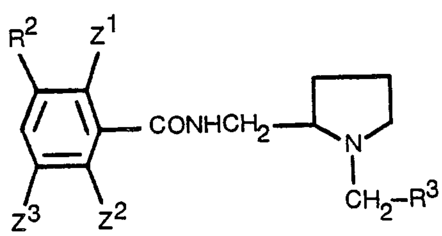

wherein $Z^1$, $Z^2$, $Z^3$, $R^2$ and $R^3$ are as defined in the preamble of the claim and at least one of $Z^1$ and $Z^3$ is OH, with a compound of the formula

$$R^1—X^3$$

wherein $R^1$ has the definition given in the preamble of the claim and $X^3$ is a leaving group, to the formation of a compound of the formula

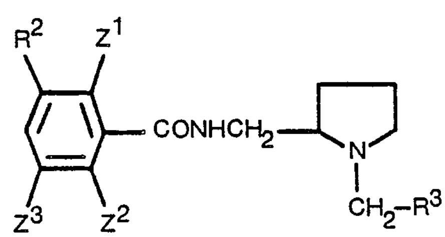

wherein $Z^1$, $Z^2$, $Z^3$, $R^2$ and $R^3$ are as defined in the preamble of the claim and at least one of $Z^1$ and $Z^3$ is $OR^1$, whereafter, if desired, the compound obtained by any of methods a) — h) is converted to a physiologically acceptable salt thereof and/or converted to a substantially pure stereoisomer thereof.

4. A process according to claim 3 characterized in that a compound according to any of claims 1—2 is prepared.

5. An intermediate for the preparation of a compound of the formula I of claim 1 having the formula

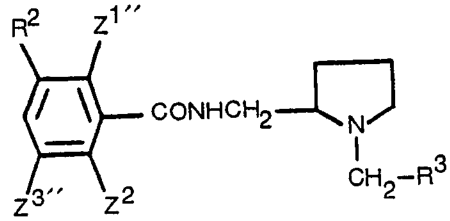

wherein $Z^2$, $R^2$ and $R^3$ are as defined in claim 1 and $Z^{1''}$ is OH, $OR^1$ or a suitably protected hydroxy group and $Z^{3''}$ is OH, $OR^4$, $OR^1$ or a suitably protected hydroxy group and at least one of $Z^{1''}$ and $Z^{3''}$ is a suitably protected hydroxy group.

6. A compound of the formula

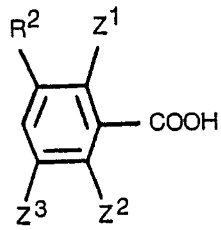

II

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C—(CH_2)_n—$,

$R^1$ is alkyl CO, wherein alkyl represents a straight or branched hydrocarbon chain having 1 to 17 carbon atoms,

$R^4$ is an alkyl group of 1—4 carbon atoms.

7. A pharmaceutical preparation comprising as active ingredient a compound according to any of claims 1—2 or a physiologically acceptable salt or an optical isomer thereof.

8. A pharmaceutical preparation according to claim 7 in dosage unit form.

9. A pharmaceutical preparation according to claims 7—8 comprising the active ingredient in association with a pharmaceutically acceptable carrier.

10. The use of a compound according to any of claims 1—2 or a physiologically acceptable salt thereof for the preparation of a pharmaceutical preparation comprising as an active ingredient an amount of said compound.

11. A compound according to any of claims 1—2 for use as a drug for the treatment of diseases related to a dysfunction of the dopaminergic system.

**Claims for the Contracting State: AT**

1. A process for the preparation of a compound of the formula

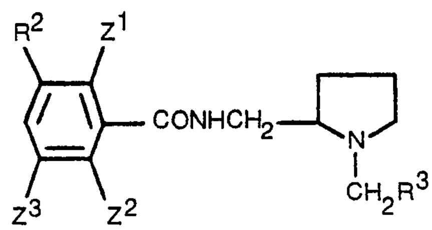

I

wherein

$Z^1$ is OH or $OR^1$,

$Z^2$ is $OR^4$,

$Z^3$ is OH, $OR^4$ or $OR^1$,

$R^2$ is a hydrogen atom, a halogen atom, $R^4$ or $F_3C—(CH_2)_n—$

$R^3$ is a hydrogen atom, $R^4$, a straight or branched hydrocarbon chain with a double or triple bond and 2 to 3 carbon atoms, phenyl or phenyl substituted with methylenedioxy or one or more of fluoro, chloro, bromo, trifluoromethyl, methyl, ethyl, methoxy or ethoxy in the orto, meta or para positions,

$R^1$ is alkyl CO, wherein alkyl represents a straight or branched hydrocarbon chain having 1 to 17 carbon atoms,

$R^4$ is an alkyl group of 1—4 carbon atoms,

n is 0, 1 or 2,

or a physiologically acceptable salt or optical isomer thereof, which process comprises

a) reaction of a compound of the formula

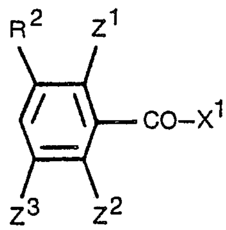

wherein $Z^1$, $Z^2$, $Z^3$ and $R^2$ have the above given definitions and $—CO—X^1$ is a reactive group capable of reacting with an amino group under formation of an amide moiety, with a compound of the formula

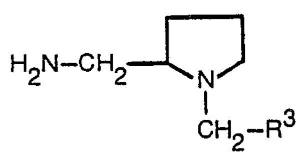

wherein $R^3$ has the above given definition, or a reactive derivative thereof, to the formation of a compound of the formula I, or

b) N-substitution of a compound of the formula

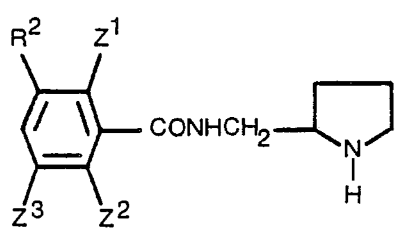

wherein $R^2$, $Z^1$, $Z^2$ and $Z^3$ have the definitions given in the preamble of the claim, with a compound of the formula

$$R^3—CH_2—X^2$$

wherein $R^3$ has the definition given in the preamble of the claim and $X^2$ is a leaving group, to the formation of a compound of the formula I, or

c) reduction of a compound of the formula

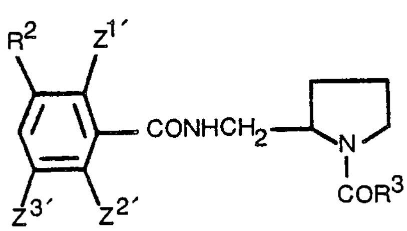

wherein $R^2$, $R^3$, $Z^{1'}$, $Z^{2'}$ and $Z^{3'}$ have the definitions given below, to the formation of a compound of the formula

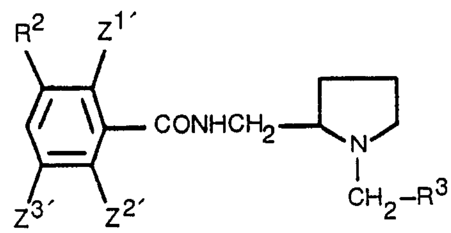

wherein $R^2$ and $R^3$ are as defined in the preamble of the claim and $Z^{1'}$ is OH, $Z^{2'}$ is $OR^4$ and $Z^{3'}$ is OH or $OR^4$, or

d) deprotection of a compound of the formula

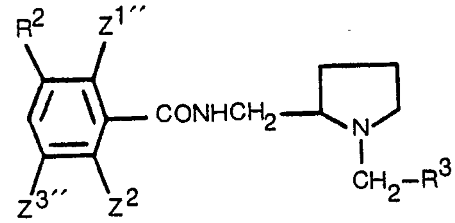

wherein $Z^2$, $R^2$ and $R^3$ are as defined in the preamble of the claim, $Z^{1''}$ is OH, $OR^1$ or a suitably protected hydroxy group and $Z^{3''}$ is OH, $OR^4$, $OR^1$ or a suitably protected hydroxy group and at least one of $Z^{1''}$ and $Z^{3''}$ is a suitably protected hydroxy group, to the formation of a compound of a formula

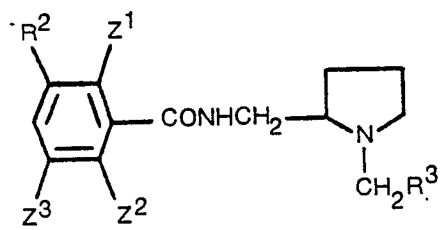

wherein $Z^1$, $Z^2$, $Z^3$, $R^2$ and $R^3$ are as defined in the preamble of the claim and at least one of $Z^1$ and $Z^3$ is OH, or

e) reaction of a compound of the formula

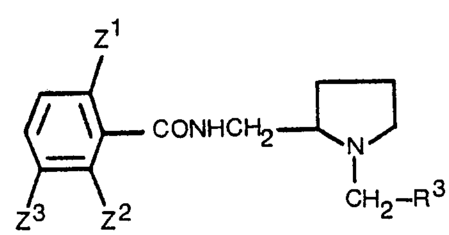

wherein $Z^1$, $Z^2$, $Z^3$ and $R^3$ are as defined in the preamble of the claim, with a halogenating agent, to the formation of a compound of the formula

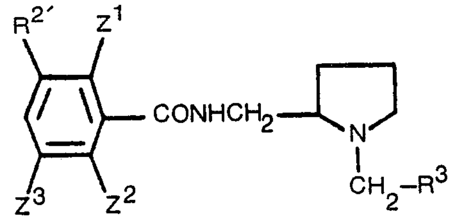

wherein $Z^1$, $Z^2$, $Z^3$ and $R^3$ are as defined in the preamble of the claim and $R^{2'}$ is Br or Cl, or

f) reaction of a compound of the formula

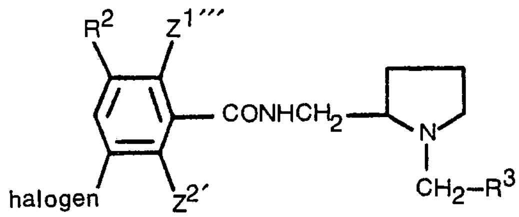

wherein $R^2$ and $R^3$ are as defined in the preamble of the claim, $Z^{1'''}$ and $Z^{2'}$ is $OR^4$ and halogen is Cl, Br or I, with potassium hydroxide or sodium hydroxide in aqueous media, to the formation of a compound of the formula

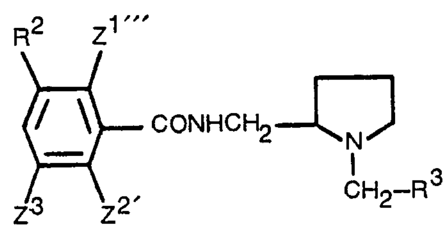

wherein $Z^3$, $R^2$ and $R^3$ are as defined in the preamble of the claim and $Z^{1'''}$ and $Z^{2'}$ is $OR^4$, or

g) catalytic hydrogenation of a compound of the formula

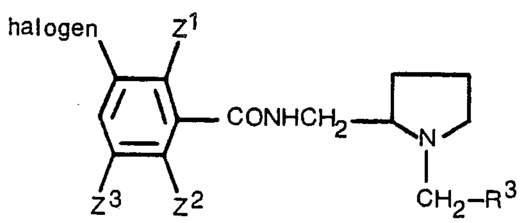

wherein $Z^1$, $Z^2$, $Z^3$ and $R^3$ are as defined in the preamble of the claim and halogen is Cl, Br or I, to the formation of a compound of the formula

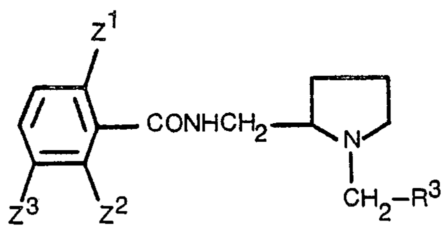

wherein $Z^1$, $Z^2$, $Z^3$ and $R^3$ are as defined in the preamble of the claim, or

h) reaction of a compound of the formula

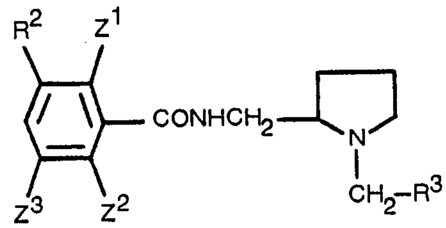

wherein $Z^1$, $Z^2$, $Z^3$, $R^2$ and $R^3$ are as defined in the preamble of the claim and at least one of $Z^1$ and $Z^3$ is OH, with a compound of the formula

$$R^1—X^3$$

wherein $R^1$ has the definition given in the preamble of the claim and $X^3$ is a leaving group, to the formation of a compound of the formula

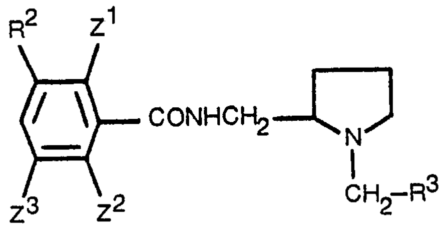

wherein $Z^1$, $Z^2$, $Z^3$, $R^2$ and $R^3$ are as defined in the preamble of the claim and at least one of $Z^1$ and $Z^3$ is $OR^1$, whereafter, if desired, the compound obtained by any of methods a) — h) is converted to a physiologically acceptable salt thereof and/or converted to a substantially pure stereoisomer thereof.

2. A process according to claim 1 characterized in that a compound of the formula

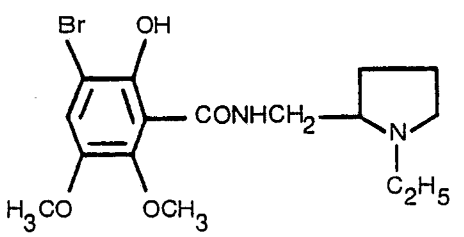

or

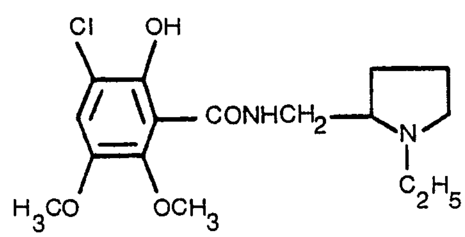

or

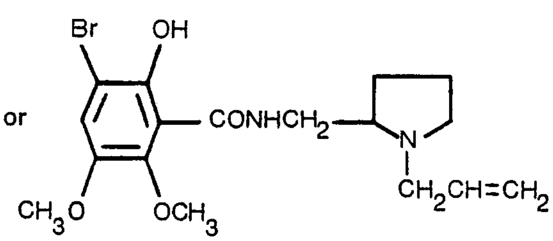

or

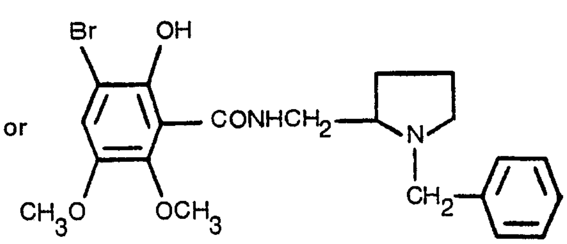

or

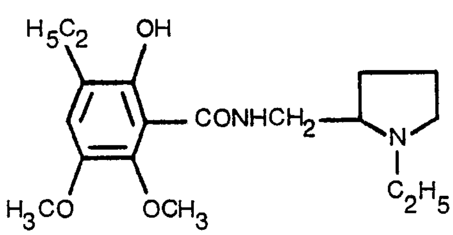

or

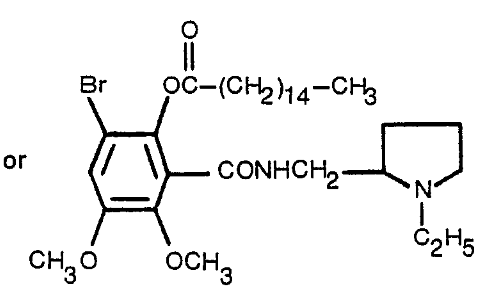

is prepared.

**Patentansprüche für die Vertragsstaaten: BE CH DE FR GB IT LI LU NL SE**

1. Verbindung der Formel

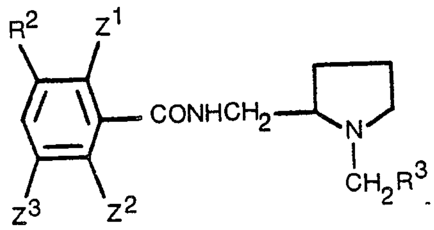

I

worin

$Z^1$ OH oder $OR^1$ ist,

$Z^2$ $OR^4$ ist,

$Z^3$ OH, $OR^4$ oder $OR^1$ ist,

$R^2$ ein Wasserstoffatom, ein Halogenatom, $R^4$ oder $F_3C-(CH_2)_n$ darstellt,

$R^3$ ein Wasserstoffatom, $R^4$, eine gerade oder verzweigte Kohlenwasserstoffkette mit einer Doppel- oder Dreifachbindung und 2 bis 3 Kohlenstoffatomen, Phenyl oder durch Methylendioxy oder einem oder mehreren von Fluor, Chlor, Brom, Trifluormethyl, Methyl, Ethyl, Methoxy oder Ethoxy in der Ortho-, Meta- oder Para-Position substituiertes Phenyl bedeutet,

$R^1$ Alkyl-CO ist, worin Alkyl für eine gerade oder verzweigte Kohlenwasserstoffkette mit 1 bis 17 Kohlenstoffatomen steht,

$R^4$ eine Alkylgruppe mit 1—4 Kohlenstoffatomen ist,

n für 0, 1 oder 2 steht,

oder ein physiologisch akzeptables Salz oder optisches Isomeres davon.

2. Verbindung nach Anspruch 1 der Formel

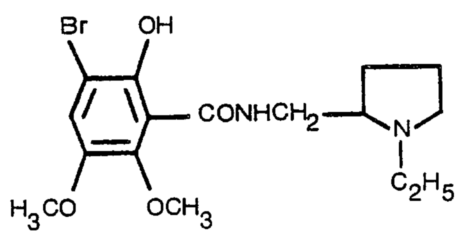

oder

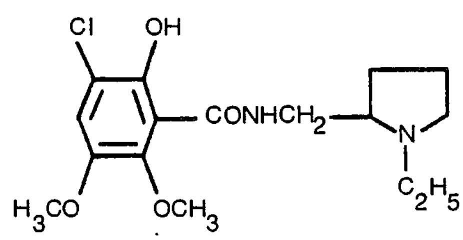

oder

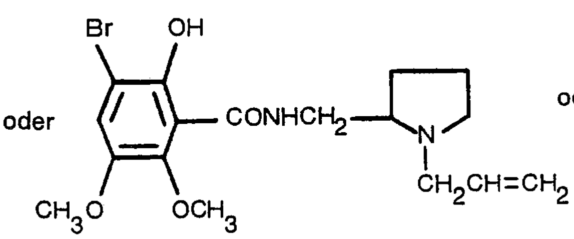

oder

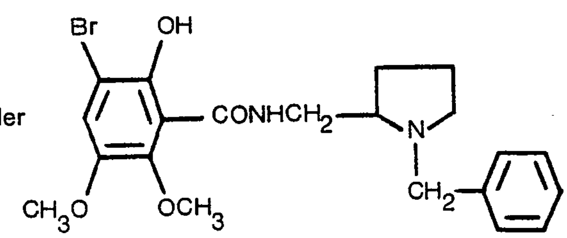

oder

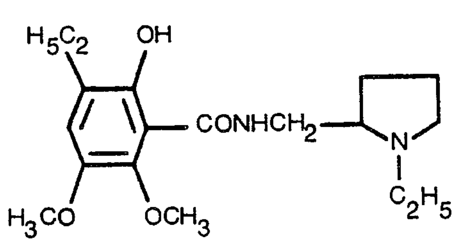

oder

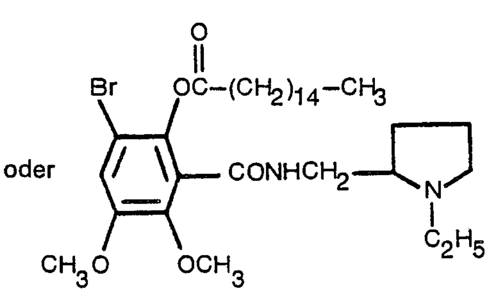

3. Verfahren zur Herstellung einer Verbindung der Formel

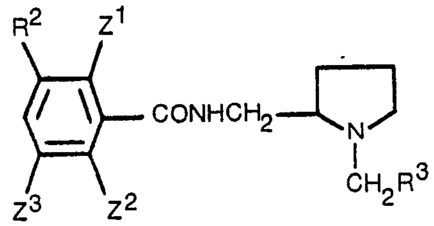

I

worin
$Z^1$ OH oder $OR^1$ ist,
$Z^2$ $OR^4$ ist,
$Z^3$ OH, $OR^4$ oder $OR^1$ ist,
$R^2$ ein Wasserstoffatom, ein Halogenatom, $R^4$ oder $F_3C—(CH_2)_n$ darstellt,
$R^3$ ein Wasserstoffatom, $R^4$, eine gerade oder verzweigte Kohlenwasserstoffkette mit einer Doppel- oder Dreifachbindung und 2 bis 3 Kohlenstoffatomen, Phenyl oder durch Methylendioxy oder einem oder mehreren von Fluor, Chlor, Brom, Trifluormethyl, Methyl, Ethyl, Methoxy oder Ethoxy in der Ortho-, Meta- oder Para-Position substituiertes Phenyl bedeutet,
$R^1$ Alkyl-CO ist, worin Alkyl für eine gerade oder verzweigte Kohlenwasserstoffkette mit 1 bis 17 Kohlenstoffatomen steht,
$R^4$ eine Alkylgruppe mit 1—4 Kohlenstoffatomen ist,
n für 0, 1 oder 2 steht,
oder eines physiologisch akzeptablen Salzes davon, welches Verfahren umfaßt:
a) die Umsetzung einer Verbindung der Formel

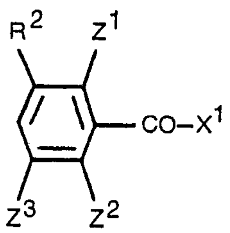

worin $Z^1$, $Z^2$, $Z^3$ und $R^2$ die oben angeführten Bedeutungen haben und $—CO—X^1$ eine reaktive, mit einer Aminogruppe unter Bildung eines Amidanteils reaktionsfähige Gruppe darstellt, mit einer Verbindung der Formel

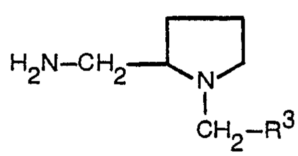

worin $R^3$ die oben angeführte Bedeutung hat, oder eines reaktiven Derivats davon, zur Bildung einer Verbindung der Formel I, oder
b) die N-Substitution einer Verbindung der Formel

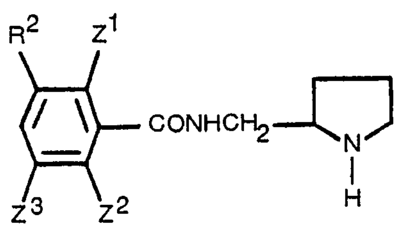

worin $R^2$, $Z^1$, $Z^2$ und $Z^3$ die im Oberbegriff des Anspruchs angeführten Bedeutungen haben, mit einer Verbindung der Formel

$R^3—CH_2—X_2$

worin $R^3$ die im Oberbegriff des Anspruchs angeführte Bedeutung hat und $X^2$ eine austretende Gruppe darstellt, zur Bildung einer Verbindung der Formel I, oder

c) die Reduktion einer Verbindung der Formel

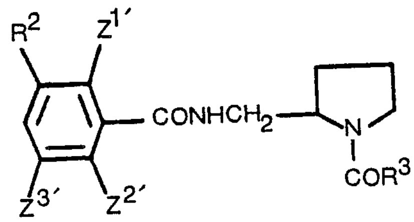

worin $R^2$, $R^3$, $Z^{1'}$, $Z^{2'}$ und $Z^{3'}$ die nachstehenden Bedeutungen haben, zur Bildung einer Verbindung der Formel

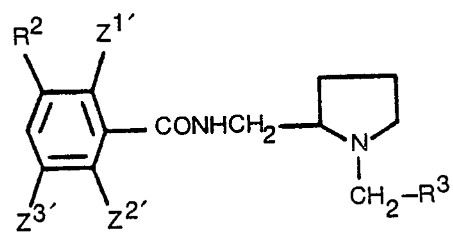

worin $R^2$ und $R^3$ wie im Oberbegriff des Anspruchs definiert sind und $Z^{1'}$ für OH steht, $Z^{2'}$ $OR^4$ ist und $Z^{3'}$ OH oder $OR^4$ bedeutet, oder

d) die Entschützung einer Verbindung der Formel

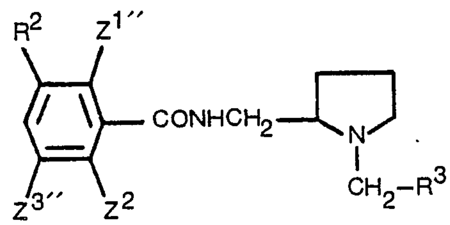

worin $Z^2$, $R^2$ und $R^3$ wie im Oberbegriff des Anspruchs definiert sind, $Z^{1''}$ für OH, $OR^1$ oder eine zweckmäßig geschützte Hydroxygruppe steht und $Z^{3''}$ OH, $OR^4$, $OR^1$ oder eine zweckmäßig geschützte Hydroxygruppe darstellt, und zumindest eines von $Z^{1''}$ und $Z^{3''}$ eine zweckmäßig geschützte Hydroxygruppe ist, zur Bildung einer Verbindung der Formel

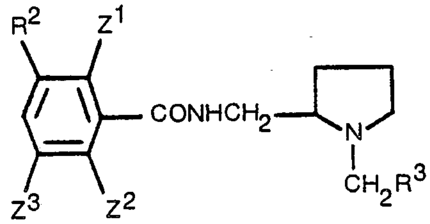

worin $Z^1$, $Z^2$, $Z^3$, $R^2$ und $R^3$ wie im Oberbegriff des Anspruchs definiert sind und zumindest eines von $Z^1$ und $Z^3$ für OH steht, oder

e) die Umsetzung einer Verbindung der Formel

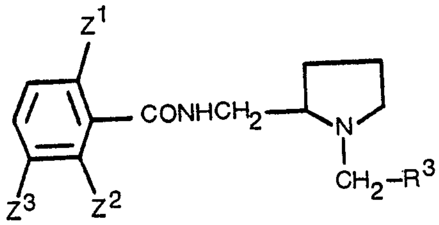

worin $Z^1$, $Z^2$, $Z^3$ und $R^3$ wie im Oberbegriff des Anspruchs definiert sind, mit einem Halogenierungsmittel zur Bildung einer Verbindung der Formel

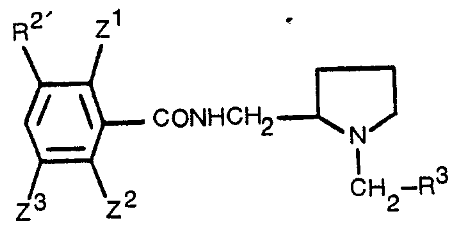

worin $Z^1$, $Z^2$, $Z^3$ und $R^3$ wie im Oberbegriff des Anspruchs definiert sind und $R^{2'}$ für Br oder Cl steht, oder

f) die Umsetzung einer Verbindung der Formel

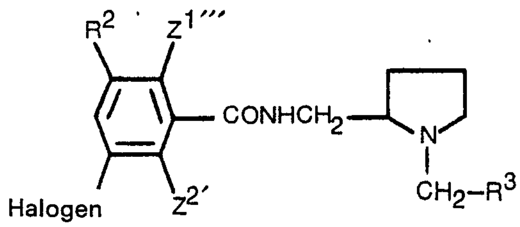

worin $R^2$ und $R^3$ wie im Oberbegriff des Anspruchs definiert sind, $Z^{1'''}$ und $Z^{2'}$ für $OR^4$ steht, und Halogen Cl, Br oder J bedeutet, mit Kaliumhydroxid oder Natriumhydroxid in wässerigen Medien zur Bildung einer Verbindung der Formel

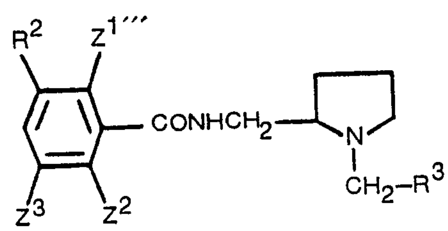

worin $Z^3$, $R^2$ und $R^3$ wie im Oberbegriff des Anspruchs definiert sind und $Z^{1'''}$ und $Z^{2'}$ $OR^4$ ist, oder

g) die katalytische Hydrierung einer Verbindung der Formel

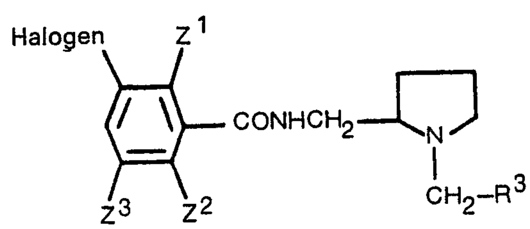

worin $Z^1$, $Z^2$, $Z^3$ und $R^3$ wie im Oberbegriff des Anspruchs definiert sind, und Halogen Cl, Br oder J bedeutet, zur Bildung einer Verbindung der Formel

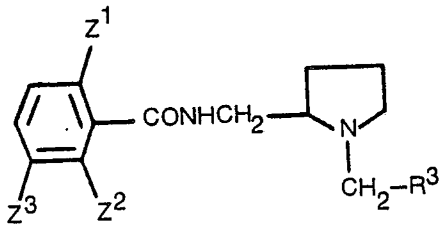

worin $Z^1$, $Z^2$, $Z^3$ und $R^3$ wie im Oberbegriff des Anspruchs definiert sind, oder

h) die Umsetzung einer Verbindung der Formel

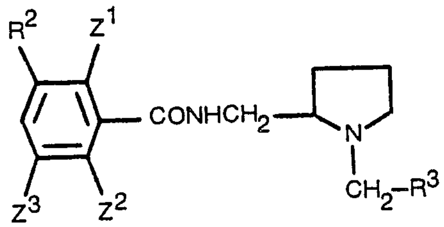

worin $Z^1$, $Z^2$, $Z^3$, $R^2$ und $R^3$ wie im Oberbegriff des Anspruchs definiert sind und zumindest eines von $Z^1$ und $Z^3$ OH ist, mit einer Verbindung der Formel

$$R^1—X^3$$

worin $R^1$ die im Oberbegriff des Anspruchs angegebene Bedeutung hat und $X^3$ eine austretende Gruppe ist, zur Bildung einer Verbindung der Formel

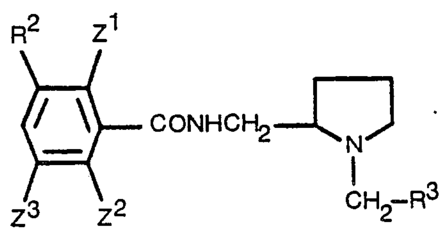

worin $Z^1$, $Z^2$, $Z^3$, $R^2$ une $R^3$ wie im Oberbegriff des Anspruchs definiert sind und zumindest eines von $Z^1$ und $Z^3$ für $OR^1$ steht,
worauf gewünschtenfalls die auf einem der Wege a)—h) erhaltene Verbindung in ein physiologisch akzeptables Salz davon und/oder in ein im wesentlichen reines Stereoisomere davon übergeführt wird.

4. Verfahren nach Anspruch 3, dadurch gekennzeichnet, daß eine Verbindung nach einem der Ansprüche 1—2 hergestellt wird.

5. Zwischenprodukt zur Herstellung einer Verbindung der Formel I des Anspruchs 1 mit der Formel

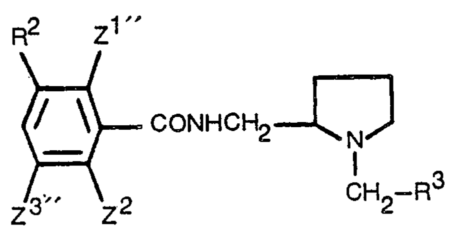

worin $Z^2$, $R^2$ und $R^3$ wie im Anspruch 1 definiert sind und $Z^{1''}$ OH, $OR^1$ oder eine zweckmäßig geschützte Hydroxygruppe ist, und $Z^{3''}$ für OH, $OR^4$, $OR^1$ oder eine zweckmäßig geschützte Hydroxygruppe steht und

zumindest eines von $Z^{1''}$ und $Z^{3''}$ eine zweckmäßig geschützte Hydroxygruppe ist.

6. Verbindung der Formel

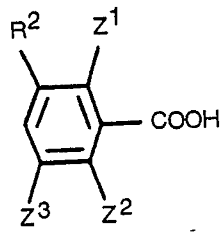

II

worin

$Z^1$ OH oder $OR^1$ ist,

$Z^2$ für $OR^4$ steht,

$Z^3$ OH, $OR^4$ oder $OR^1$ ist,

$R^2$ ein Wasserstoffatom, ein Halogenatom, $R^4$ oder $F_3C—(CH_2)_n$-darstellt,

$R^1$ Alkyl-CO ist, worin Alkyl eine gerade oder verzweigte Kohlenwasserstoffkette mit 1 bis 17 Kohlenstoffatomen bedeutet,

$R^4$ eine Alkylgruppe mit 1—4 Kohlenstoffatomen ist.

7. Pharmazeutisches Präparat, das als aktives Ingrediens eine Verbindung nach einem der Ansprüche 1—2 oder ein physiologisch akzeptables Salz oder ein optisches Isomeres davon umfaßt.

8. Pharmazeutisches Präparat nach Anspruch 7 in Dosiseinheitsform.

9. Pharmazeutisches Präparat nach den Ansprüchen 7—8, welches das aktive Ingrediens in Verbindung mit einem pharmazeutisch akzeptablen Träger umfaßt.

10. Verwendung einer Verbindung nach einem der Ansprüche 1—2 oder eines physiologisch akzeptablen Salzes davon zur Herstellung eines pharmazeutischen Präparats, das als aktives Ingrediens eine Menge dieser Verbindung enthält.

11. Verbindung nach einem der Ansprüche 1—2 zur Verwendung als Arznei bei der Behandlung von Erkrankungen, die mit Funktionsstörungen des dopaminergischen Systems verbunden sind.

**Patentansprüche für den Vertragsstaat: AT**

1. Verfahren zur Herstellung einer Verbindung der Formel

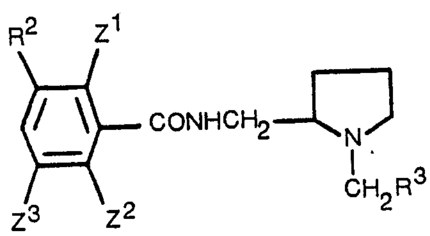

I

worin

$Z^1$ OH oder $OR^1$ ist,

$Z^2$ $OR^4$ ist,

$Z^3$ OH, $OR^4$ oder $OR^1$ ist,

$R^2$ ein Wasserstoffatom, ein Halogenatom, $R^4$ oder $F_3C—(CH_2)_n$ darstellt,

$R^3$ ein Wasserstoffatom, $R^4$, eine gerade oder verzweigte Kohlenwasserstoffkette mit einer Doppel- oder Dreifachbindung und 2 bis 3 Kohlenstoffatomen, Phenyl oder durch Methylendioxy oder einem oder mehreren von Fluor, Chlor, Brom, Trifluormethyl, Methyl, Ethyl, Methoxy oder Ethoxy in der Ortho-, Meta- oder Para-Position substituiertes Phenyl bedeutet,

$R^1$ Alkyl-CO ist, worin Alkyl für eine gerade oder verzweigte Kohlenwasserstoffkette mit 1 bis 17 Kohlenstoffatomen steht,

$R^4$ eine Alkylgruppe mit 1—4 Kohlenstoffatomen ist,

n für 0, 1 oder 2 steht,

oder eines physiologisch akzeptablen Salzes davon, welches Verfahren umfaßt:

a) die Umsetzung einer Verbindung der Formel

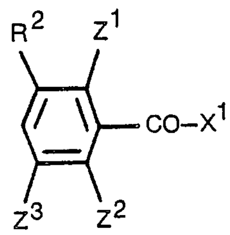

worin $Z^1$, $Z^2$, $Z^3$ und $R^2$ die oben angeführten Bedeutungen haben und —CO—$X^1$ eine reaktive, mit einer Aminogruppe unter Bildung eines Amidanteils reaktionsfähige Gruppe darstellt, mit einer Verbindung der Formel

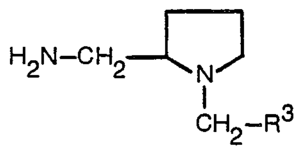

worin $R^3$ die oben angeführte Bedeutung hat, oder eines reaktiven Derivats davon, zur Bildung einer Verbindung der Formel I, oder

b) die N-Substitution einer Verbindung der Formel

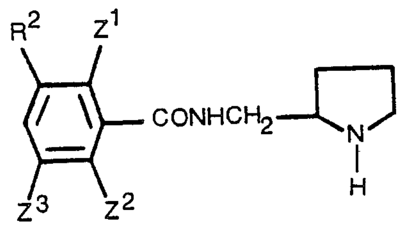

worin $R^2$, $Z^1$, $Z^2$ und $Z^3$ die im Oberbegriff des Anspruchs angeführten Bedeutungen haben, mit einer Verbindung der Formel

$$R^3—CH_2—X_2$$

worin $R^3$ die im Oberbegriff des Anspruchs angeführte Bedeutung hat und $X^2$ eine austretende Gruppe darstellt, zur Bildung einer Verbindung der Formel I, oder

c) die Reduktion einer Verbindung der Formel

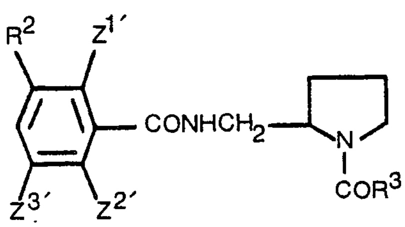

worin $R^2$, $R^3$, $Z^{1'}$, $Z^{2'}$ und $Z^{3'}$ die nachstehenden Bedeutungen haben, zur Bildung einer Verbindung der Formel

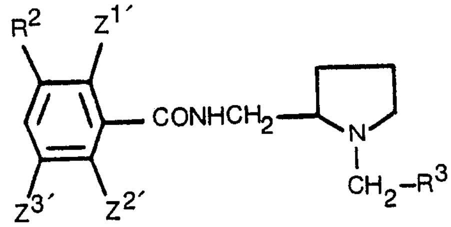

worin $R^2$ und $R^3$ wie im Oberbegriff des Anspruchs definiert sind und $Z^{1'}$ für OH steht, $Z^{2'}$ $OR^4$ ist und $Z^{3'}$ OH oder $OR^4$ bedeutet, oder

d) die Entschützung einer Verbindung der Formel

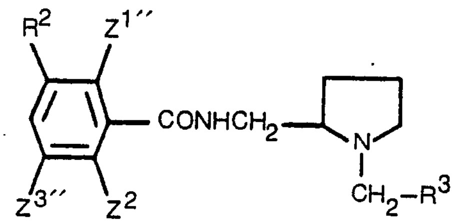

worin $Z^2$, $R^2$ und $R^3$ wie im Oberbegriff des Anspruchs definiert sind, $Z^{1''}$ für OH, $OR^1$ oder eine zweckmäßig geschützte Hydroxygruppe steht und $Z^{3''}$ OH, $OR^4$, $OR^1$ oder eine zweckmäßig geschützte Hydroxygruppe darstellt, und zumindest eines von $Z^{1''}$ und $Z^{3''}$ eine zweckmäßig geschützte Hydroxygruppe ist, zur Bildung einer Verbindung der Formel

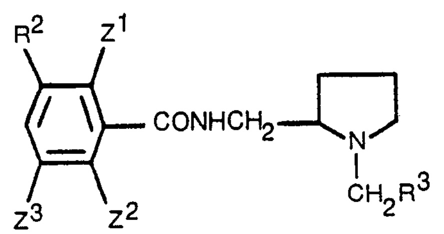

worin $Z^1$, $Z^2$, $Z^3$, $R^2$ und $R^3$ wie im Oberbegriff des Anspruchs definiert sind und zumindest eines von $Z^1$ und $Z^3$ für OH steht, oder

e) die Umsetzung einer Verbindung der Formel

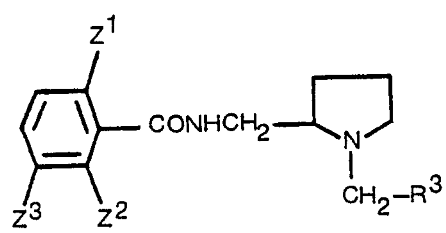

worin $Z^1$, $Z^2$, $Z^3$ und $R^3$ wie im Oberbegriff des Anspruchs definiert sind, mit einem Halogenierungsmittel zur Bildung einer Verbindung der Formel

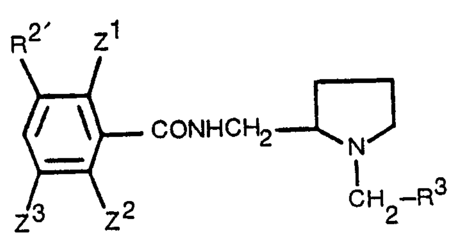

worin $Z^1$, $Z^2$, $Z^3$ und $R^3$ wie im Oberbegriff des Anspruchs definiert sind und $R^{2'}$ für Br oder Cl steht, oder

f) die Umsetzung einer Verbindung der Formel

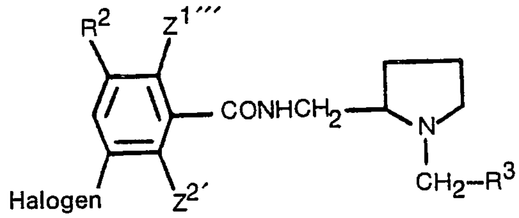

worin $R^2$ und $R^3$ wie im Oberbegriff des Anspruchs definiert sind, $Z^{1'''}$ und $Z^{2'}$ für $OR^4$ steht, und Halogen Cl, Br oder J bedeutet, mit Kaliumhydroxid oder Natriumhydroxid in wässerigen Medien zur Bildung einer Verbindung der Formel

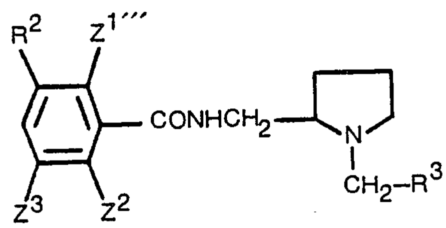

worin $Z^3$, $R^2$ und $R^3$ wie im Oberbegriff des Anspruchs definiert sind und $Z^{1'''}$ und $Z^{2'}$ $OR^4$ ist, oder

g) die katalytische Hydrierung einer Verbindung der Formel

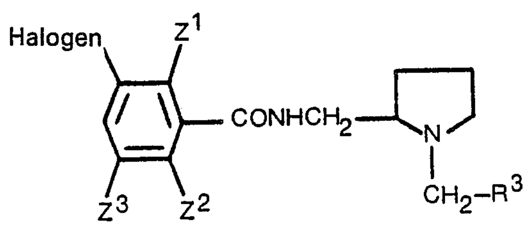

worin $Z^1$, $Z^2$, $Z^3$ und $R^3$ wie im Oberbegriff des Anspruchs definiert sind, und Halogen Cl, Br oder J bedeutet, zur Bildung einer Verbindung der Formel

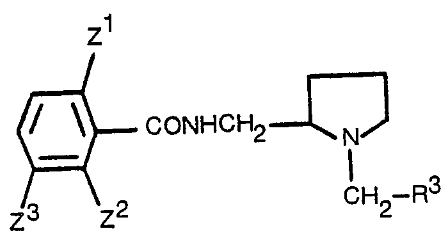

worin $Z^1$, $Z^2$, $Z^3$ und $R^3$ wie im Oberbegriff des Anspruchs definiert sind, oder

h) die Umsetzung einer Verbindung der Formel

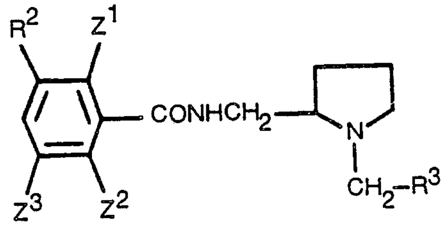

worin $Z^1$, $Z^2$, $Z^3$, $R^2$ und $R^3$ wie im Oberbegriff des Anspruchs definiert sind und zumindest eines von $Z^1$ und $Z^3$ OH ist, mit einer Verbindung der Formel

$$R^1—X^3$$

worin $R^1$ die im Oberbegriff des Anspruchs angegebene Bedeutung hat und $X^3$ eine austretende Gruppe ist, zur Bildung einer Verbindung der Formel

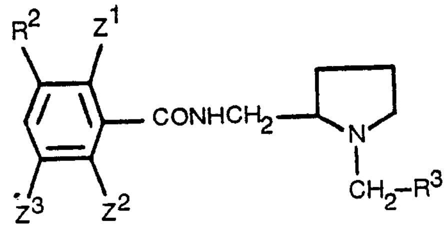

worin $Z^1$, $Z^2$, $Z^3$, $R^2$ und $R^3$ wie im Oberbegriff des Anspruchs definiert sind und zumindest eines von $Z^1$ und $Z^3$ für $OR^1$ steht, worauf gewünschtenfalls die auf einem der Wege a)—h) erhaltene Verbindung in ein physiologisch akzeptables Salz davon und/oder in ein im wesentlichen reines Stereoisomere davon übergeführt wird.

2. Verfahren nach Anspruch 1, dadurch gekennzeichnet, daß eine Verbindung der Formel

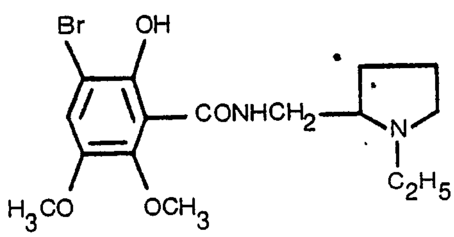

oder

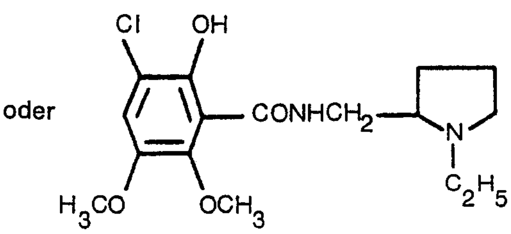

oder

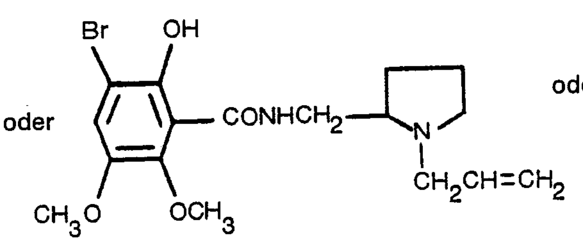

oder

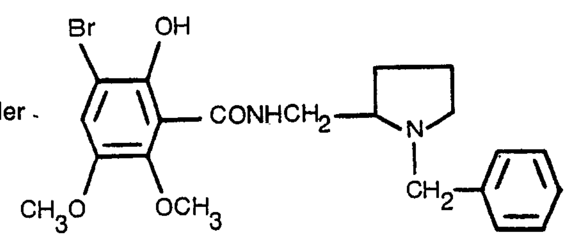

oder

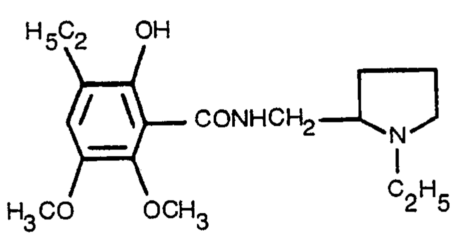

oder

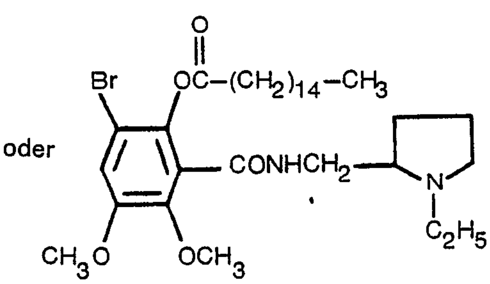

hergestellt wird.

**Revendications pour les Etats contractants: BE CH DE FR IT LI LU NL SE**

1. Un composé de formule

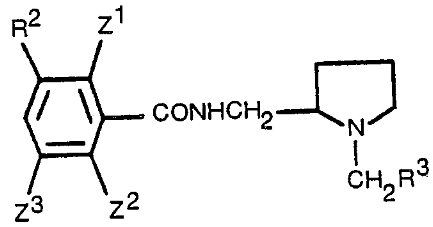

I

dans laquelle

$Z^1$ est OH ou $OR^1$,

$Z^2$ est $OR^4$,

$Z^3$ est OH, $OR^4$ ou $OR^1$,

$R^2$ est un atome d'hydrogène, un atome d'halogène, $R^4$ ou $F_3C—(CH_2)_n—$,

$R^3$ est un atome d'hydrogène, $R^4$, une chaîne hydrocarbonée droite ou ramifiée avec une double ou une triple liaison et 2 à 3 atomes de carbone, un phényle ou un phényle substitué par un méthylènedioxy ou par un ou plusieurs des fluoro, chloro, bromo, trifluorométhyle, méthyle, éthyle, méthoxy ou éthoxy dans les positions ortho, méta ou para,

$R^1$ est un alkyl-CO où l'alkyle représente une chaîne hydrocarbonée droite ou ramifiée avec 1 à 17 atomes de carbone,

$R^4$ est un groupe alkyle de 1 à 4 atomes de carbone,

n est 0, 1 ou 2,

ou un de ses sels physiologiquement acceptables ou un de ses isomères optiques.

2. Composé selon la revendication 1 de formule

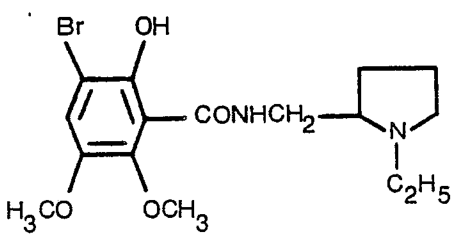

ou

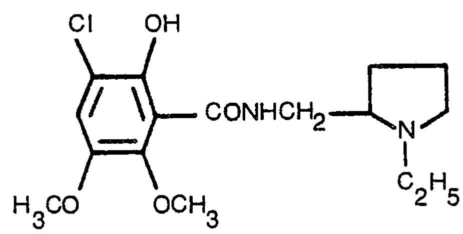

ou

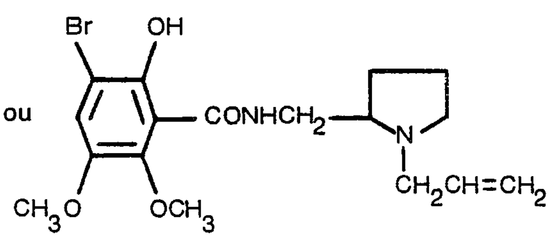

ou

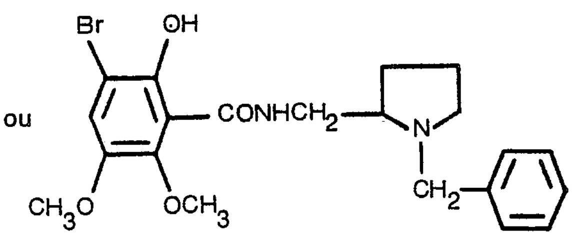

ou

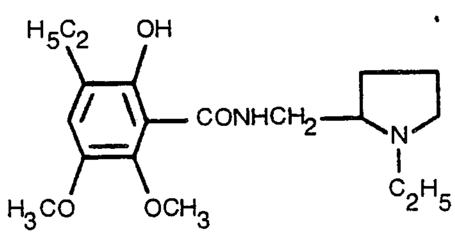

ou

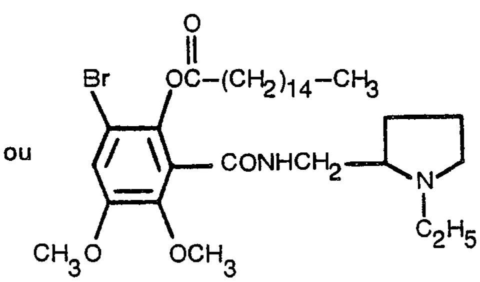

3. Un procédé pour la préparation d'un composé de formule

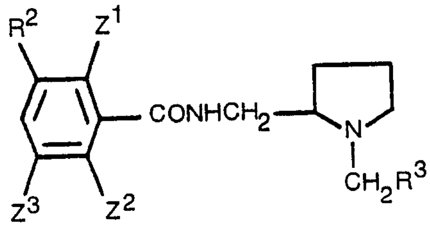

I

dans laquelle

$Z^1$ est OH ou $OR^1$,

$Z^2$ est $OR^4$,

$Z^3$ est OH, $OR^4$ ou $OR^1$,

$R^2$ est un atome d'hydrogène, un atome d'halogène, $R^4$ ou $F_3C—(CH_2)_n—$,

$R^3$ est un atome d'hydrogène, $R^4$, une chaîne hydrocarbonée droite ou ramifiée avec une double ou une triple liaison et 2 à 3 atomes de carbone, un phényle ou un phényle substitué par un méthylènedioxy ou par un ou plusieurs des fluoro, chloro, bromo, trifluorométhyle, méthyle, éthyle, méthoxy ou éthoxy dans les positions ortho, méta ou para,

$R^1$ est un alkyl-CO où l'alkyle représente une chaîne hydrocarbonée droite ou ramifiée avec 1 à 17 atomes de carbone,

$R^4$ est un groupe alkyle de 1 à 4 atomes de carbone,

n est 0, 1 ou 2,

ou d'un de ses sels physiologiquement acceptables ou d'un de ses isomères optiques, lequel procédé comprend

a) la réaction d'un composé de formule

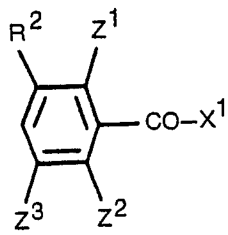

dans laquelle $Z^1$, $Z^2$, $Z^3$ et $R^2$ répondent aux définitions données ci-dessus et $—CO—X^1$ est un groupe réactif capable de réagir avec un groupe amino en formant un groupement amido, avec un composé de formule

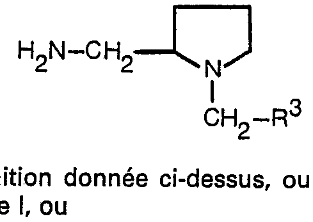

dans laquelle $R^3$ répond a la définition donnée ci-dessus, ou un de ses dérivés réactifs, en vue de la formation d'un composé de formule I, ou

b) la N-substitution d'un composé de formule

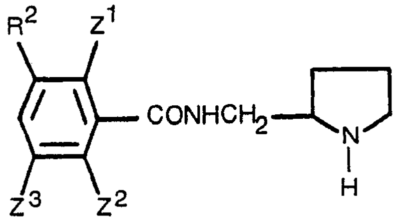

dans laquelle $R^2$, $Z^1$, $Z^2$ et $Z^3$ répondent aux définitions données dans le préambule de la revendication, avec un composé de formule

$R^3—CH_2—X^2$

dans laquelle $R^3$ répond à la définition donnée dans le préambule de la revendication et $X^2$ est un groupe partant, en vue de la formation d'un composé de formule I, ou

c) la réduction d'un composé de formule

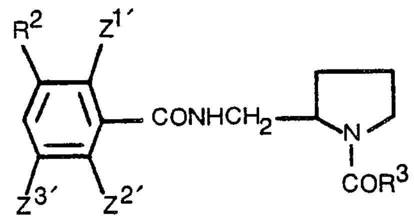

dans laquelle $R^2$, $R^3$, $Z^{1'}$, $Z^{2'}$ et $Z^{3'}$ répondent aux définitions données ci-dessous, en vue de la formation d'un composé de formule

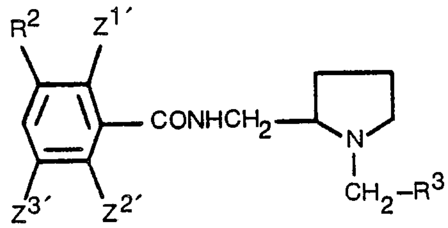

dans laquelle $R^2$ et $R^3$ sont tels que définis dans le préambule de la revendication et $Z^{1'}$ est OH, $Z^{2'}$ est $OR^4$ et $Z^{3'}$ est OH ou $OR^4$, ou

d) la déprotection d'un composé de formule

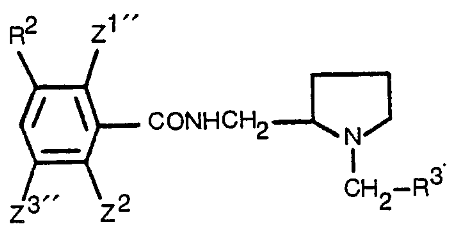

dans laquelle $Z^2$, $R^2$ et $R^3$ sont tels que définis dans le préambule de la revendication, $Z^{1''}$ est OH, $OR^1$ ou un groupe hydroxyle convenablement protégé et $Z^{3''}$ est OH, $OR^4$, $OR^1$ ou un groupe hydroxyle convenablement protégé et au moins un des $Z^{1''}$ et $Z^{3''}$ est un groupe hydroxyle convenablement protégé, en vue de la formation d'un composé de formule

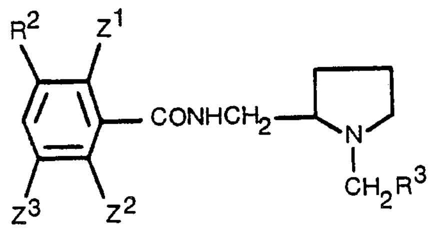

dans laquelle $Z^1$, $Z^2$, $Z^3$, $R^2$ et $R^3$ sont tels que définis dans le préambule de la revendication et au moins un des $Z^1$ et $Z^3$ est OH, ou

e) la réaction d'un composé de formule

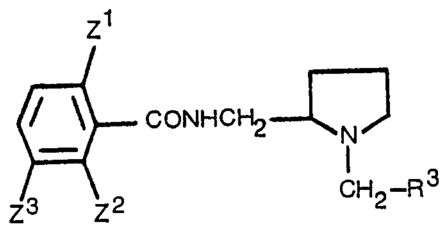

dans laquelle $Z^1$, $Z^2$, $Z^3$ et $R^3$ sont tels que définis dans le préambule de la revendication, avec un agent d'halogénation, en vue de la formation d'un composé de formule

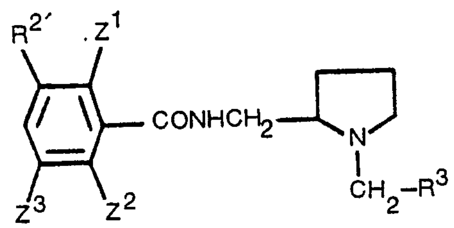

dans laquelle $Z^1$, $Z^2$, $Z^3$ et $R^3$ sont tels que définis dans le préambule de la revendication et $R^{2'}$ est Br ou Cl, ou

f) la réaction d'un composé de formule

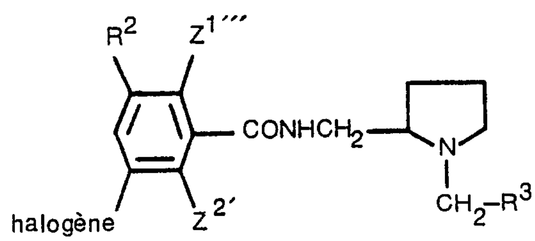

dans laquelle $R^2$ et $R^3$ sont tels que définis dans le préambule de la revendication, $Z^{1'''}$ et $Z^{2'}$ sont $OR^4$ et l'halogène est Cl, Br ou I, avec de l'hydroxyde de potassium ou de l'hydroxyde de sodium en milieu aqueux, en vue de la formation d'un composé de formule

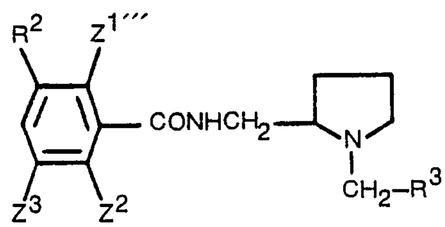

dans laquelle $Z^3$, $R^2$ et $R^3$ sont tels que définis dans le préambule de la revendication et $Z^{1'''}$ et $Z^{2'}$ sont $OR^4$, ou

g) l'hydrogénation catalytique d'un composé de formule

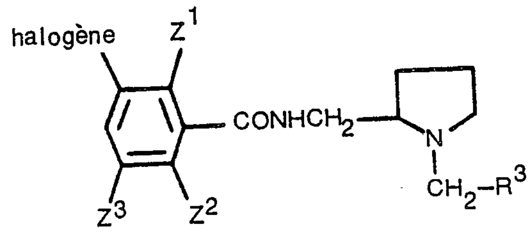

dans laquelle $Z^1$, $Z^2$, $Z^3$ et $R^3$ sont tels que définis dans le préambule de la revendication et l'halogène est Cl, Br ou I, en vue de la formation d'un composé de formule

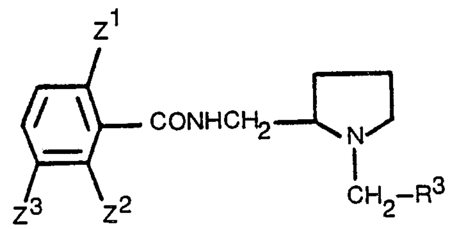

dans laquelle $Z^1$, $Z^2$, $Z^3$ et $R^3$ sont tels que définis dans le préambule de la revendication, ou

h) la réaction d'un composé de formule

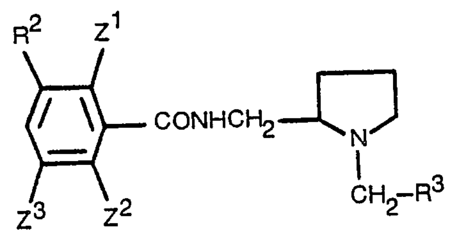

dans laquelle $Z^1$, $Z^2$, $Z^3$, $R^2$ et $R^3$ sont tels que définis dans le préambule de la revendication et au moins un des $Z^1$ et $Z^3$ est OH, avec un composé de formule

$$R^1—X^3$$

dans laquelle $R^1$ répond à la définition donnée dans le préambule de la revendication et $X^3$ est un groupe partant, en vue de la formation d'un composé de formule

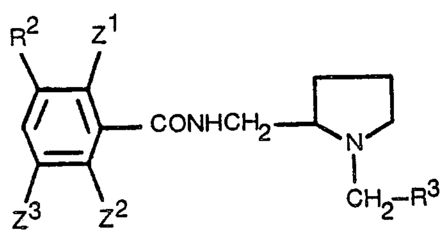

dans laquelle $Z^1$, $Z^2$, $Z^3$, $R^2$ et $R^3$ sont tels que définis dans le préambule de la revendication et au moins un des $Z^1$ et $Z^3$ est $OR^1$, après quoi, si on le désire, le composé obtenu par l'une des méthodes a) à h) est transformé en un de ses sels physiologiquement acceptables et/ou transformé en un de ses stéréoisomères essentiellement purs.

4. Procédé selon la revendication 3, caractérisé en ce que l'on prépare un composé selon l'une des revendications 1 à 2.

5. Un produit intermédiaire pour la préparation d'un composé de formule I de la revendication 1, répondant à la formule

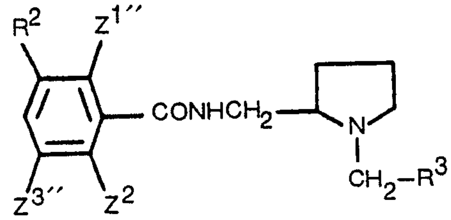

dans laquelle $Z^2$, $R^2$ et $R^3$ sont tels que définis dans la revendication 1 et $Z^{1''}$ est OH, $OR^1$ ou un groupe hydroxyle convenablement protégé et $Z^{3''}$ est OH, $OR^4$, $OR^1$ ou un groupe hydroxyle convenablement protégé.et au moins un des $Z^{1''}$ et $Z^{3''}$ est un groupe hydroxyle convenablement protége.

6. Un composé de formule

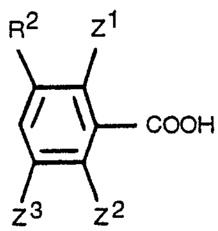

dans laquelle

$Z^1$ est OH ou $OR^1$,

$Z^2$ est $OR^4$,

$Z^3$ est OH, $OR^4$ ou $OR^1$,

$R^2$ est un atome d'hydrogène, un atome d'halogène, $R^4$ ou $F_3C—(CH_2)_n—$,

$R^1$ est un alkyl-CO où l'alkyle représente une chaîne hydrocarbonée droite ou ramifiée ayant 1 à 17 atomes de carbone,

$R^4$ est un groupe alkyle de 1 à 4 atomes de carbone.

7. Une préparation pharmaceutique contenant, à titre de constituant actif, un composé selon l'une des revendications 1 à 2, un de ses sels physiologiquement acceptables ou un de ses isomères optiques.

8. Préparation pharmaceutique selon la revendication 7, sous forme de dose unitaire.

9. Préparation pharmaceutique selon les revendications 7 à 8 contenant le constituant actif en association avec un excipient pharmaceutiquement acceptable.

10. Emploi d'un composé selon l'une quelconque des revendications 1 à 2 ou d'un de sels physiologiquement acceptables pour la confection d'une préparation pharmaceutique contenant, à titre de constituant actif, une quantité dudit composé.

11. Un composé selon l'une des revendications 1 à 2, destiné à l'emploi comme médicament pour le traitement des affections liées au dysfonctionnement du système dopaminergique.

**Revendications pour l'Etat contractant: AT**

1. Un procédé pour la préparation d'un composé de formule

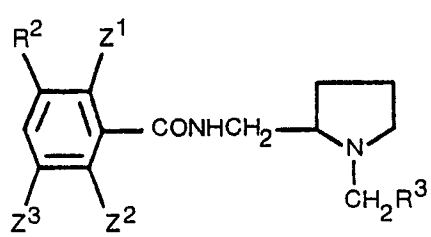

I

dans laquelle

$Z^1$ est OH ou $OR^1$,

$Z^2$ est $OR^4$,

$Z^3$ est OH, $OR^4$ ou $OR^1$,

$R^2$ est un atome d'hydrogène, un atome d'halogène, $R^4$ ou $F_3C—(CH_2)_n—$,

$R^3$ est un atome d'hydrogène, $R^4$, une chaîne hydrocarbonée droite ou ramifiée avec une double ou une triple liaison et 2 à 3 atomes de carbone, un phényle ou un phényle substitué par un méthylènedioxy ou par un ou plusieurs des fluoro, chloro, bromo, trifluorométhyle, méthyle, éthyle, méthoxy ou éthoxy dans les positions ortho, méta ou para,

$R^1$ est un alkyl-CO où l'alkyle représente une chaîne hydrocarbonée droite ou ramifiée avec 1 à 17 atomes de carbone,

$R^4$ est un groupe alkyle de 1 à 4 atomes de carbone,

n est 0, 1 ou 2,

ou d'un de ses sels physiologiquement acceptables ou d'un de ses isomères optiques, lequel procédé comprend

a) la réaction d'un composé de formule

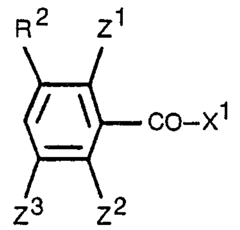

dans laquelle $Z^1$, $Z^2$, $Z^3$ et $R^2$ répondent aux définitions données ci-dessus et $—CO—X^1$ est un groupe réactif capable de réagir avec un groupe amino en formant un groupement amido, avec un composé de formule

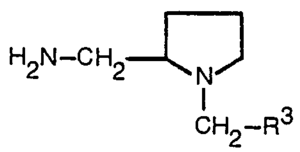

dans laquelle $R^3$ répond a la définition donnée ci-dessus, ou un de ses dérivés réactifs, en vue de la formation d'un composé de formule I, ou

b) la N-substitution d'un composé de formule

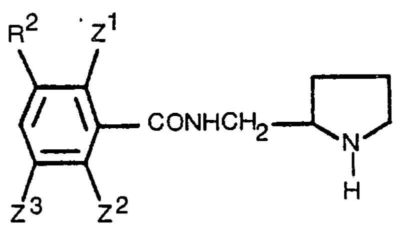

dans laquelle $R^2$, $Z^1$, $Z^2$ et $Z^3$ répondent aux définitions données dans le préambule de la revendication, avec un composé de formule

$$R^3—CH_2—X^2$$

dans laquelle $R^3$ répond à la définition donnée dans le préambule de la revendication et $X^2$ est un groupe partant, en vue de la formation d'un composé de formule I, ou

c) la réduction d'un composé de formule

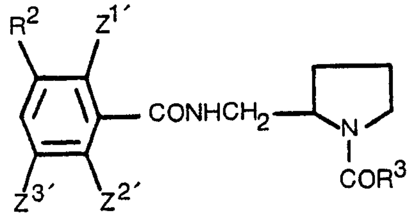

dans laquelle $R^2$, $R^3$, $Z^{1'}$, $Z^{2'}$ et $Z^{3'}$ répondent aux définitions données ci-dessous, en vue de la formation d'un composé de formule

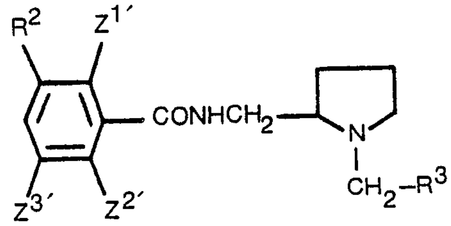

dans laquelle $R^2$ et $R^3$ sont tels que définis dans le préambule de la revendication et $Z^{1'}$ est OH, $Z^{2'}$ est $OR^4$ et $Z^{3'}$ est OH ou $OR^4$, ou

d) la déprotection d'un composé de formule

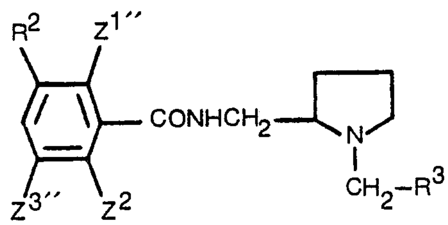

dans laquelle $Z^2$, $R^2$ et $R^3$ sont tels que définis dans le préambule de la revendication, $Z^{1''}$ est OH, $OR^1$ ou un groupe hydroxyle convenablement protégé et $Z^{3''}$ est OH, $OR^4$, $OR^1$ ou un groupe hydroxyle convenablement protégé et au moins un des $Z^{1''}$ et $Z^{3''}$ est un groupe hydroxyle convenablement protégé, en vue de la formation d'un composé de formule

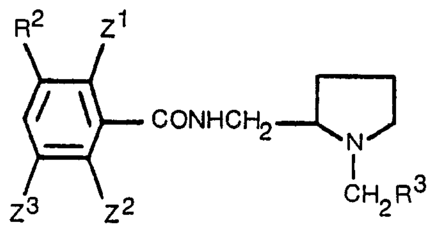

dans laquelle $Z^1$, $Z^2$, $Z^3$, $R^2$ et $R^3$ sont tels que définis dans le préambule de la revendication et au moins un des $Z^1$ et $Z^3$ est OH, ou

e) la réaction d'un composé de formule

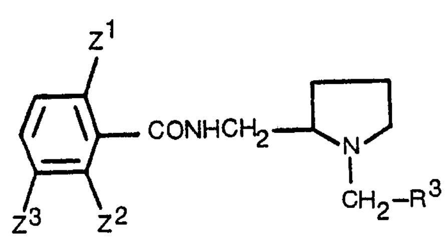

dans laquelle $Z^1$, $Z^2$, $Z^3$ et $R^3$ sont tels que définis dans le préambule de la revendication, avec un agent d'halogénation, en vue de la formation d'un composé de formule

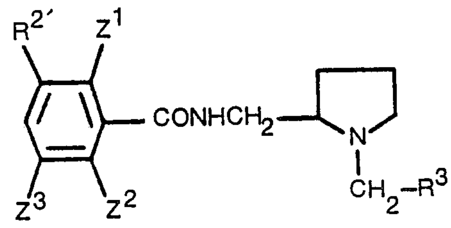

dans laquelle $Z^1$, $Z^2$, $Z^3$ et $R^3$ sont tels que définis dans le préambule de la revendication et $R^{2'}$ est Br ou Cl, ou

f) la réaction d'un composé de formule

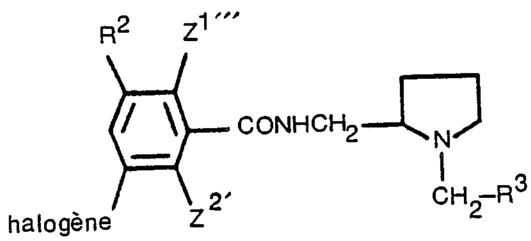

dans laquelle $R^2$ et $R^3$ sont tels que définis dans le préambule de la revendication, $Z^{1'''}$ et $Z^{2'}$ sont $OR^4$ et l'halogène est Cl, Br ou I, avec de l'hydroxyde de potassium ou de l'hydroxyde de sodium en milieu aqueux, en vue de la formation d'un composé de formule

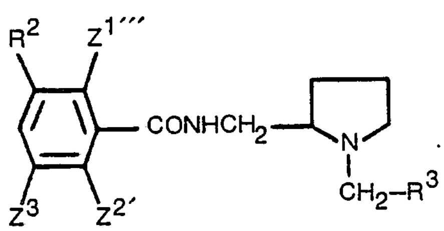

dans laquelle $Z^3$, $R^2$ et $R^3$ sont tels que définis dans le préambule de la revendication et $Z^{1'''}$ et $Z^{2'}$ sont $OR^4$, ou

g) l'hydrogénation catalytique d'un composé de formule

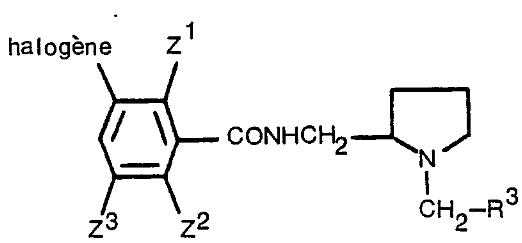

dans laquelle $Z^1$, $Z^2$, $Z^3$ et $R^3$ sont tels que définis dans le préambule de la revendication et l'halogène est Cl, Br ou I, en vue de la formation d'un composé de formule

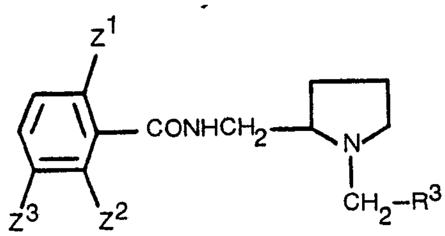

dans laquelle $Z^1$, $Z^2$, $Z^3$ et $R^3$ sont tels que définis dans le préambule de la revendication, ou

h) la réaction d'un composé de formule

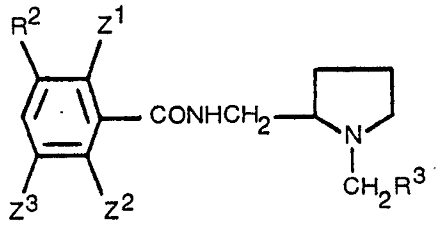

dans laquelle $Z^1$, $Z^2$, $Z^3$, $R^2$ et $R^3$ sont tels que définis dans le préambule de la revendication et au moins un des $Z^1$ et $Z^3$ est OH, avec un composé de formule

$$R^1{-}X^3$$

dans laquelle $R^1$ répond à la définition donnée dans le préambule de la revendication et $X^3$ est un groupe partant, en vue de la formation d'un composé de formule

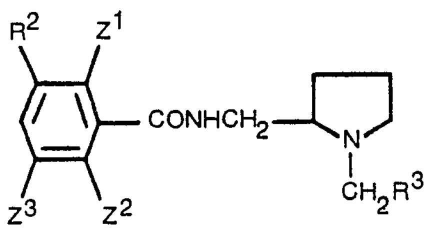

dans laquelle $Z^1$, $Z^2$, $Z^3$, $R^2$ et $R^3$ sont tels que définis dans le préambule de la revendication et au moins un des $Z^1$ et $Z^3$ est $OR^1$, après quoi, si on le désire, le composé obtenu par l'une des méthodes a) à h) est transformé en un de ses sels physiologiquement acceptables et/ou transformé en un de ses stéréoisomères essentiellement purs.

2. Un procédé selon la revendication 1, caractérisé en ce que l'on prépare un composé de formule

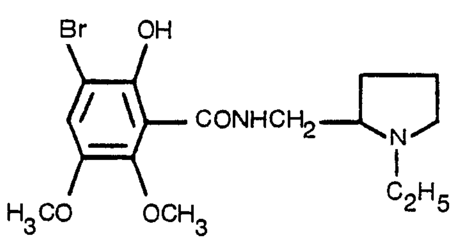

ou

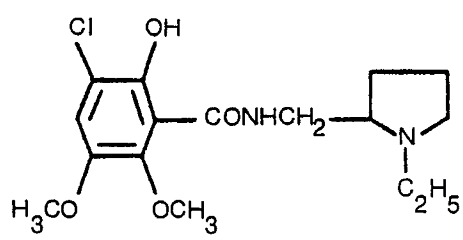

ou

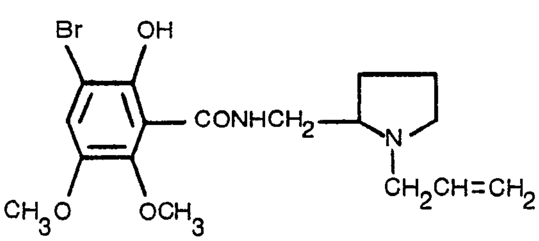

ou

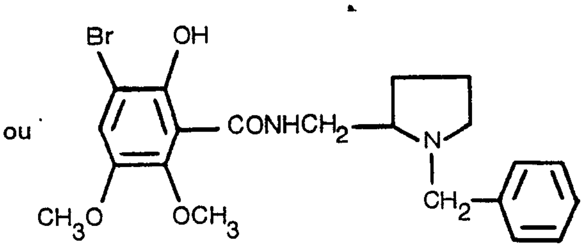

ou 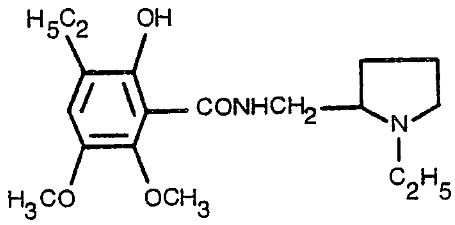 ou